# EUROPÄISCHE PATENTSCHRIFT

(11) **EP 3 245 937 B1**
(45) Veröffentlichungstag und Bekanntmachung des Hinweises auf die Patenterteilung: **06.05.2020**
(21) Anmeldenummer: 17171499.1
(22) Anmeldetag: 17.05.2017
(51) Int. Cl.: A61B 1/05, A61B 1/12, A61B 1/313

(54) **ENDOSKOP UND REINIGUNGSINSTRUMENT FÜR EIN ENDOSKOP**
ENDOSCOPE AND CLEANING INSTRUMENT FOR AN ENDOSCOPE
ENDOSCOPE ET INSTRUMENT DE NETTOYAGE POUR UN ENDOSCOPE

(30) Priorität: 17.05.2016 DE 102016109066
(43) Veröffentlichungstag der Anmeldung: 22.11.2017
(73) Patentinhaber: Karl Storz SE & Co. KG, 78532 Tuttlingen (DE)
(72) Erfinder: Irion, Klaus, 78532 Tuttlingen (DE); Bacher, Uwe, 78532 Tuttlingen (DE); Sauer, Michael, 78532 Tuttlingen (DE)
(74) Vertreter: Wimmer, Stephan

(56) Entgegenhaltungen:
- US-A- 5 334 150
- US-A1- 2003 093 088
- US-A1- 2004 102 804
- US-A1- 2014 066 711
- US-A1- 2015 080 933
- US-A1- 2015 157 311

## Beschreibung

Die vorliegende Erfindung ist auf die Reinigung der Lichteintrittsfläche am distalen Ende eines Endoskops in situ bezogen, insbesondere auf ein Endoskop und ein Reinigungsinstrument, die eine derartige Reinigung ermöglichen.

Während mikroinvasiver Maßnahmen können an der äußeren Oberfläche eines Endoskops und damit auch an der Lichteintrittsfläche am distalen Ende des Endoskops feste und flüssige Ablagerungen entstehen. Diese können durch einen ungewollten unmittelbaren Kontakt mit einer Gewebeoberfläche, durch Blut oder eine andere Körperflüssigkeit, die beispielsweise an einer Wunde austritt, oder kleine Gewebepartikel, die bei der Maßnahme freigesetzt und umher geschleudert werden, entstehen. Ferner kann Feuchtigkeit aus der in der Regel nahezu gesättigten Atmosphäre an der Lichteintrittsfläche des Endoskops kondensieren.

Eine Ablagerung an einer Lichteintrittsfläche eines Endoskops kann eine Abschattung, Streuung, Brechung oder Beugung von Licht und damit eine erhebliche Verschlechterung der Bildqualität bewirken. Auch eine Ablagerung an einer Lichtaustrittsfläche, durch die Beleuchtungslicht am distalen Ende eines Endoskops austreten soll, verschlechtert die Bildqualität in der Regel, indem sie die Intensität des Beleuchtungslichts im zu beobachtenden Bereich reduziert, das Beleuchtungslicht filtert und sein Spektrum verändert. Ferner kann eine Ablagerung an einem Grenzbereich zwischen einer Lichtaustrittsfläche und einer Lichteintrittsfläche eine unmittelbare Einkopplung von Beleuchtungslicht in Beobachtungsstrahlengang bewirken.

Wenn keine andere apparative Vorkehrungen getroffen sind, muss ein verschmutztes Endoskop während der mikrochirurgischen Maßnahme herausgezogen, gereinigt und wieder eingeführt werden. Dies kostet Zeit und stört den Ablauf der mikrochirurgischen Maßnahme. Ferner kann eine Unterbrechung der mikrochirurgischen Maßnahme oder ihrer Sichtkontrolle in bestimmten Phasen ein erhebliches Risiko für den Patienten bergen. Hinzu kommt, dass die Lichteintrittsfläche des Endoskops bei der Reinigung außerhalb des Körpers abkühlen kann, wodurch das Risiko einer nachfolgenden Kondensation von Feuchtigkeit steigt. Wenn ein Endoskop durch einen Halte- oder Roboterarm gehalten wird, ist seine Reinigung noch aufwendiger.

In EP 1 210 904 A2, EP 1 323 373 A2, US 5,207,213, US 5,392,766 und EP 2 111 782 A2 sind verschiedene Maßnahmen zur Reinigung der Lichtaus- und -eintrittsflächen am distalen Ende eines Endoskops in situ beschrieben. Diese Maßnahmen erfordern eigenen Bauraum. Dadurch wird der Querschnitt des Endoskopschafts und/oder der Querschnitt des Lumens eines Trokartubus vergrößert.

In EP 0 434 793 B1 ist ein Endoskop mit einer am distalen Ende angeordneten Videoeinheit 1 beschrieben. Die Videoeinheit 1 ist schwenkbar angeordnet und kann während des Einführens des Endoskops durch einen Trokartubus hindurch innerhalb der Kontur des Endoskops und während einer mikroinvasiven und/oder minimalinvasiven Maßnahme teilweise oder vollständig außerhalb der Kontur des Endoskops angeordnet sein (Figuren 1a, 1b, 1e, 6c, 6b, 6a). Ferner ist ein Endoskop beschrieben, bei dem eine Videoeinheit 1 innerhalb eines Hauptkanals 4 eines Schafts 2 des Endoskops längs verschiebbar ist (Absatz [0075], Figuren 8a, 8b). Wenn die Videoeinheit 1 am Operationsort aus dem Hauptkanal 4 des Schafts 2 des Endoskops nach distal herausgeschoben wird, kann sie einen Teil des Kanalquerschnitts freigeben (ebd.).

Ähnliche Endoskope sind in WO 2015/029040 und CH 698 893 B1 beschrieben.

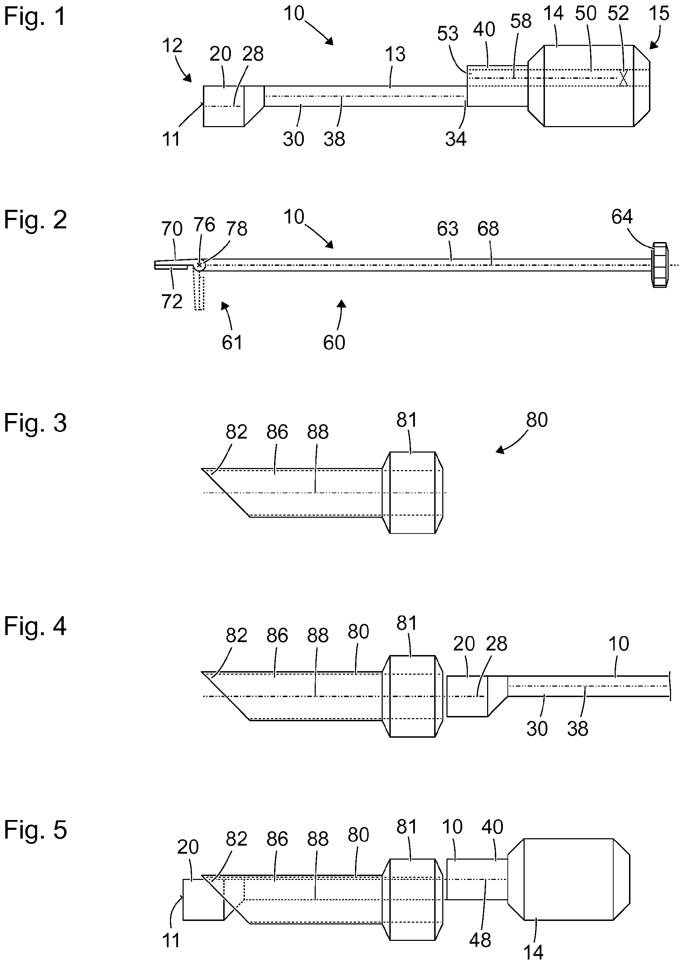

In US 2009/0231419 A1 ist eine Haupt-Endoskop ("main endoscope") 10 mit einem Längskanal ("longitudinal channel") 22 beschrieben (Absätze [0048], [0051], Figur 3). In dem Längskanal 22 ist ein kleines Endoskop ("minor endoscope") 20 mit einer weiteren Bilderfassungsvorrichtung 42 angeordnet (ebd.). Das kleine Endoskop 20 weist einen flexiblen Abschnitt ("flexible link") 44 mit reduziertem Querschnitt auf (Absätze [0057], [0076], Figur 3).

In US 6,387,043 B1 ist ein Endoskop beschrieben, das als Trokar ("penetrating member") 12 mit einem schneidenden und optisch transparenten distalen Ende 22 ausgebildet ist (Spalte 4, Zeilen 54 bis 67; Figur 3). In Figur 3 ist ein länglicher rohrartiger Körper 18 mit einem kleineren Querschnitt dargestellt als das distale Ende 22 und das proximale Ende 24.

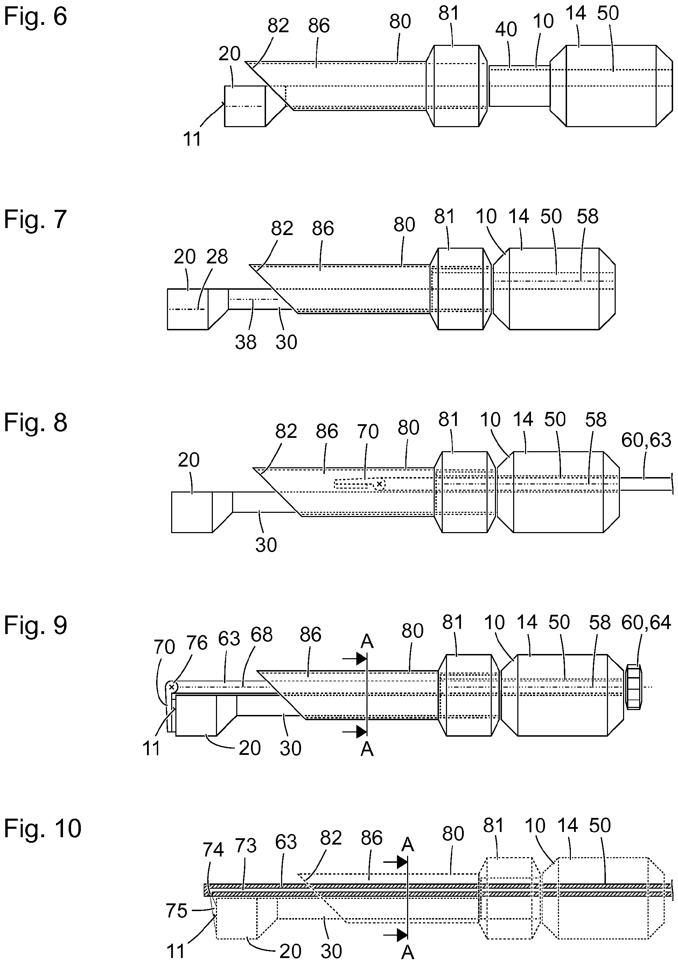

In US 4,867,138 ist ein starres elektronisches Endoskop 1 beschrieben, das in einer Hülle 2 angeordnet werden kann (Spalte 3, Zeilen 52 bis 54; Figur 1). Das Endoskop 1 weist eine Spitze 31 mit einem größeren Durchmesser und einen sich proximal anschließenden Teil 32 mit einem "geringeren Durchmesser" auf (Figuren 1, 2). Der Querschnitt des Teils 32 mit "geringerem Durchmesser" kann halbkreisförmig mit einem geraden Randabschnitt (Figur 4) oder mit Ausnehmungen 61 (Figur 5) oder mit überstehenden Kanten 62 (Figur 6) oder elliptisch (Figur 7; tatsächlich mit kreisbogenförmigen und geraden Randabschnitten) oder meniskusförmig (Figur 8) sein (Spalte 6, Zeilen 10 bis 68).

In US 5,514,084 ist eine zurückziehbarer Wischer zum Reinigen eines endoskopischen chirurgischen Instruments beschrieben. Das endoskopische chirurgische Instrument und der zurückziehbare Wischer werden durch zwei verschiedene Öffnungen eingeführt (Figur 1).

In US 2015/0080933 A1 ist ein medizinisches Werkzeug 534 zur Verwendung in einem Hohlraum eines Patienten beschrieben (Absatz [0149], Figuren 5A, 5B). Das Werkzeug 534 umfasst mehrere Kameras 546a, 546b und mehrere Lichtquellen 550a, 550b (ebd.). Seitlich an dem Werkzeug 534 ist ein Ende eines Trägers 230 zum Bewegen des Werkzeugs 534 in einem Arbeitskanal angeordnet (ebd.). Der Träger 230 kann in einer Führung 632 in einer äußeren Hülle 642 eines Katheters geführt werden (Absätze [0155] bis [0157]; Figuren 6A bis 6F).

In US 2015/0157311 A1 ist eine Laparoskopievorrichtung zur Verankerung eines Organs am Zwerchfell (thoracic diaphragm) beschrieben (Absatz [0001]). Die Laparoskopievorrichtung ist vorgesehen, um eine Befestigungsanordnung 1 in den Bauchraum AC einzuführen und mit dieser die Gallenblase GB am Zwerchfell D zu befestigen (Absatz [0059], Figur 1). Die Befestigungsanordnung umfasst ein T-förmiges Ende 1, das durch eine Öffnung in die Gallenblase GB einzuführen ist, und einen Anker A mit Fluken 6 zur Befestigung am Zwerchfell (Absatz [0060], Figur 2). Der Anker A ist durch einen Faden 3 mit dem T-förmigen Ende 1 verbunden (ebd.). Die Laparoskopievorrichtung umfasst eine Einführhülle 13 in Gestalt einer hohlen rohrförmigen Struktur, eine Einführnadel 14, in deren scharfem und hohlem distalem Ende 14A das T-förmige Ende 1 der Befestigungsanordnung ruht, und einen Schieber ("pusher") 15 in der Einführnadel 14, um nach dem Durchstoßen der Gallenblasenwand das T-förmige Ende 1 der Befestigungsanordnung aus dem Ende der Einführnadel 14 auszuwerfen (Absatz [0062], Figur 4). Das proximale Ende der rohrförmigen Einführhülle 13 ist zu einer zylindrischen Scheibe ("cylindrical disc") 13B erweitert (ebd.). Eine Unterlegscheibe ("washer") 18 mit zwei Öffnungen 19, 20 bedeckt die zylindrische Scheibe 13B und verhindert das Austreten von Gas aus dem Bauchraum (ebd.).

US 2004/0102804 (Figur 1B) offenbart ein Endoskop mit einen versetzten proximalem Schaftabschnitt oder Griff, jedoch ist die Kamera auch am proximalen Ende.

Bewegbare, insbesondere relativ zueinander schwenkbare oder verschiebbare Teile eines Endoskops reduzieren die mechanische Robustheit und erhöhen das Risiko einer Beschädigung sehr erheblich. Ferner werden sowohl die Fertigung des Endoskops als auch seine Reinigung erheblich aufwendiger.

Eine Aufgabe der vorliegenden Erfindung besteht darin, ein verbessertes Endoskop, ein verbessertes Reinigungsinstrument und ein verbessertes Endoskopiesystem zu schaffen.

Diese Aufgabe wird durch die Gegenstände der unabhängigen Ansprüche gelöst. Weiterbildungen sind in den abhängigen Ansprüchen angegeben.

Ein Endoskop umfasst einen Endoskopschaft mit einem distalen Schaftabschnitt und einem mittleren Schaftabschnitt und einer Lichteintrittsfläche an dem distalen Schaftabschnitt, wobei der Querschnitt des mittleren Schaftabschnitts kleiner ist als der Querschnitt des distalen Schaftabschnitts.

Ein Endoskop umfasst einen Endoskopschaft mit einem distalen Schaftabschnitt, einem mittleren Schaftabschnitt, einem proximalen Schaftabschnitt und einer Lichteintrittsfläche an dem distalen

Schaftabschnitt, wobei der Querschnitt des mittleren Schaftabschnitts kleiner als der Querschnitt des distalen Schaftabschnitts und kleiner als der Querschnitt des proximalen Schaftabschnitts ist.

Das Endoskop ist insbesondere für mikroinvasive oder andere medizinische Anwendungen und/oder für nicht medizinische technische Anwendungen vorgesehen und ausgebildet. Das Endoskop ist insbesondere ein Videoendoskop, das mindestens eine Kamera oder mindestens ein Objektiv und mindestens einen Bildsensor in dem distalen Schaftabschnitt aufweist. Der Bildsensor wandelt ein von dem Objektiv an seiner Oberfläche erzeugtes Bild in ein Bildsignal, das analog oder digital über eine Signalleitung in dem mittleren Schaftabschnitt zu dem proximalen Ende des Endoskops übertragen werden kann.

Der Endoskopschaft ist insbesondere im Wesentlichen starr und abschnittsweise gerade. Alternativ kann der Endoskopschaft abschnittsweise oder vollständig gekrümmt oder flexibel sein. Einige der nachfolgend dargestellten Merkmale, Eigenschaften und Funktionen mögen am einfachsten oder unmittelbarsten für ein Endoskop mit einem starren und geradem Endoskopschaft verständlich oder anwendbar sein, sind jedoch auch in naheliegender Weise auf einen gekrümmten oder krümmbaren Endoskopschaft übertragbar.

Der distale Schaftabschnitt und der mittlere Schaftabschnitt sind jeweils insbesondere im Wesentlichen zylinderförmig mit kreisförmigen oder anderen Querschnitten. Zylinder sind nicht nur Kreiszylinder, sondern im mathematischen Sinne allgemeine Zylinder, also Körper, die durch Verschiebung einer beliebigen Grundfläche entlang einer Gerade (Zylinderachse) entstehen. Abweichend von einer idealen Zylinderform können der distale Schaftabschnitt und der mittlere Schaftabschnitt insbesondere jeweils Nuten, Stege oder andere Einrichtungen zum Führen oder Halten eines Instruments oder zur formschlüssigen Definition einer vorbestimmten Position eines Instruments, wie sie nachfolgend beschrieben sind, umfassen.

Der mittlere Schaftabschnitt kann, insbesondere wenn er nur eine oder mehrere Leitungen zum Übertragen von elektrischer oder optischer Leistung und eines Bildsignals enthält, einen wesentlichen kleineren Querschnitt aufweisen als der distale Schaftabschnitt.

Der größere Querschnitt des distalen Schaftabschnitts - optional mit Kamera oder Objektiv und Bildsensor - bestimmt den Querschnitt des Lumens eines Trokartubus, der erforderlich ist, um den distalen Schaftabschnitt des Endoskops durch den Trokartubus hindurch in eine Körperhöhle einführen zu können. Wenn das Endoskop vollständig in den Trokartubus eingeführt ist und der distale Schaftabschnitt den Trokartubus vollständig passiert hat, ermöglicht der kleinere Querschnitt des mittleren Schaftabschnitts, dass parallel zu dem mittleren Schaftabschnitt und an diesem vorbei ein oder mehrere weitere Instrumente durch den gleichen Trokartubus hindurch eingeführt werden können. Dies kann mit einem herkömmlichen Trokartubus möglich sein und erfordert keinerlei Bewegbarkeit des distalen Schaftabschnitts relativ zu dem mittleren Schaftabschnitt oder zu einem anderen Bestandteil des Endoskops.

Beispiele für Instrumente, die parallel zu dem mittleren Schaftabschnitt und an diesem vorbei durch den gleichen Trokartubus hindurch eingeführt werden können, sind ein Reinigungsinstrument zur Reinigung oder Trocknung der Lichteintrittsfläche an dem distalen Schaftabschnitt des Endoskops; eine Zange, ein Greifer, eine Schere zum Greifen, Halten, Quetschen, Stanzen, Schneiden oder anderen Manipulieren von Gewebe; ein Lichtleiter zur Übertragung von Beleuchtungslicht zur verbesserten und/oder strukturierteren Ausleuchtung eines zu betrachtenden Objekts; ein weiteres oder zusätzliches Endoskop, das beispielsweise eine Bildgebung im nahen, mittleren oder fernen infraroten, im sichtbaren oder im ultravioletten Wellenlängenbereich ermöglichen kann; eine Sonde zum Übertragen und Applizieren von Laserlicht oder anderem Licht hoher Leistungsdichte oder von elektrischer Leistung zum Schneiden und/oder Koagulieren oder zur anderweitigen Veränderung von Gewebe (insb. Lasertherapie, Hochfrequenzchirurgie, Elektrokauterisation, photodynamische Therapie); eine gewebediagnostische Sonde, beispielsweise basierend auf optischer Kohärenztomographie (OCT), konfokaler Mikroskopie, Spektroskopie, Ultraschall; ein optisches Projektionssystem zur (zwei- und/oder dreidimensionalen) Vermessung eines Objekts oder eines Hohlraums; ein Ballonapplikator zur Dilatation bzw. Vergrößerung bzw. Dehnung eines Gefäßes oder eines anderen Hohlraumes.

Bei einem Endoskop, wie es hier beschrieben ist, ist der distale Schaftabschnitt insbesondere mit dem mittleren Schaftabschnitt starr verbunden.

Insbesondere im Vergleich zu dem in EP 0 434 793 B1 Offenbarten kann der Entfall von Gelenken oder einer anderweitigen Bewegbarkeit des distalen Schaftabschnitts relativ zu dem mittleren Schaftabschnitt oder irgendeinem anderen Teil des Endoskops die Fertigungskosten deutlich reduzieren und die mechanische Robustheit deutlich erhöhen.

Bei einem Endoskop, wie es hier beschrieben ist, ist der distale Schaftabschnitt insbesondere gegenüber dem mittleren Schaftabschnitt seitlich versetzt.

Ein seitlicher Versatz des distalen Schaftabschnitts relativ zu dem mittleren Schaftabschnitt, d. h. ein Versatz des distalen Schaftabschnitts relativ zu dem mittleren Schaftabschnitt in einer Richtung orthogonal zu den Längsachsen des distalen Schaftabschnitts hat insbesondere zur Folge, dass der distale Schaftabschnitt gegenüber dem mittleren Schaftabschnitt nicht in allen Richtungen seitlich gleich weit übersteht.

Die Längsachse eines geraden Schaftabschnitts ist die Gerade, auf der die Mittelpunkte kreisförmiger Querschnitte des Schaftabschnitts oder die Flächenschwerpunkte nicht-kreisförmiger Querschnitte des Schaftabschnitts liegen. Im Fall eines gekrümmten mittleren Schaftabschnitts stellen Bezüge auf die Längsache des mittleren Schaftabschnitts Bezüge auf jene Gerade dar, an die sich die Kurve, auf der die Mittelpunkte oder Flächenmittelpunkte der Querschnitte des mittleren Schaftabschnitts liegen, am distalen Ende des mittleren Schaftabschnitts asymptotisch annähert.

Bei einem Endoskop, wie es hier beschrieben ist, ist bezogen auf eine Projektion in Richtung parallel zur Längsachse des mittleren Schaftabschnitts der Querschnitt des mittleren Schaftabschnitts insbesondere am Rand des Querschnitts des distalen Schaftabschnitts angeordnet.

Der distale Schaftabschnitt steht somit insbesondere in einer Richtung seitlich (d. h. orthogonal zu der Längsachse des mittleren Schaftabschnitts) nicht über. Dies kann ein Vorbeiführen eines Instruments an dem in eines Lumen eines Trokartubus angeordneten Endoskopschaft vorbei vereinfachen.

Bei einem Endoskop, wie es hier beschrieben ist, weist die Fläche des Querschnitts des mittleren Schaftabschnitts insbesondere nicht mehr als zwei Drittel oder nicht mehr als die Hälfte oder nicht mehr als ein Drittel oder nicht mehr als ein Viertel der Fläche des Querschnitts des distalen Schaftabschnitts auf.

Mit der Fläche eines Querschnitts ist der Flächeninhalt der von der äußeren Kontur des Querschnitts umschlossenen Fläche gemeint. Der Querschnitt des mittleren Schaftabschnitts kann insbesondere dann deutlich kleiner als der Querschnitt des distalen Schaftabschnitts sein, wenn der mittlere Schaftabschnitt lediglich eine oder mehrere Leitungen zum Übertragen von elektrischer und/oder optischer Leistung und Steuer- und Bildsignalen aufweist. Je kleiner der Querschnitt des mittleren Schaftabschnitts ist, desto größer ist derjenige Anteil des Querschnitts des Lumens eines Trokartubus, in den der Endoskopschaft eingesetzt ist, der für das Einführen eines oder mehrerer weiterer Instrumente verbleibt.

Bei einem Endoskop, wie es hier beschrieben ist, ist der Rand des Querschnitts des distalen Schaftabschnitt insbesondere kreisförmig und weist einen ersten Radius auf, wobei der Rand des

Querschnitts des mittleren Schaftabschnitts kreisförmig ist und einen zweiten Radius, der kleiner als der erste Radius ist, aufweist.

Der zweite Radius beträgt insbesondere näherungsweise die Hälfte (30 % bis 70 % oder 40 % bis 60 %) des ersten Radius.

Kreisförmige Querschnitte sind aus rohrförmigem Halbzeug mit kreisförmigen Querschnitten einfach und kostengünstig herstellbar und weisen ein gutes Verhältnis zwischen Masse und mechanischer Steifigkeit und Robustheit auf.

Bei einem Endoskop, wie es hier beschrieben ist, ist der Rand des Querschnitts des distalen Schaftabschnitts insbesondere kreisförmig und weist einen ersten Radius auf, wobei der Rand des Querschnitts des mittleren Schaftabschnitts einen kreisbogenförmigen Randabschnitt mit einem zweiten Radius, der gleich dem ersten Radius oder kleiner als der erste Radius ist, aufweist.

Bei einem Endoskop, wie es hier beschrieben ist, ist der Rand des Querschnitts des distalen Schaftabschnitts insbesondere kreisförmig und weist einen ersten Radius auf, wobei der Rand des Querschnitts des mittleren Schaftabschnitts zwei kreisbogenförmige Randabschnitte aufweist, wobei die Radien der kreisbogenförmigen Randabschnitte jeweils gleich dem ersten Radius oder kleiner als der erste Radius sind.

Bei einem Endoskop, wie es hier beschrieben ist, ist der Rand des Querschnitts des distalen Schaftabschnitts insbesondere kreisförmig und weist einen ersten Radius auf, wobei der Rand des Querschnitts des mittleren Schaftabschnitts zwei kreisbogenförmige Randabschnitte aufweist, wobei die Radien der kreisbogenförmigen Randabschnitte jeweils gleich dem ersten Radius oder kleiner als der erste Radius sind, und wobei die Mittelpunkte der kreisbogenförmigen Randabschnitte von einander beabstandet sind.

Insbesondere sind der Mittelpunkt des ersten kreisbogenförmigen Randabschnitts zwischen dem zweiten kreisbogenförmigen Randabschnitt und dem Mittelpunkt des zweiten kreisbogenförmigen Randabschnitts und der Mittelpunkt des zweiten kreisbogenförmigen Randabschnitts zwischen dem ersten kreisbogenförmigen Randabschnitt und dem Mittelpunkt des ersten kreisbogenförmigen Randabschnitts angeordnet.

Insbesondere ein Querschnitt des mittleren Schaftabschnitts, dessen Rand aus zwei kreisbogenförmigen Randabschnitten oder im Wesentlichen aus zwei kreisbogenförmigen Randabschnitten, deren Radien dem ersten Radius gleichen, gebildet ist, kann eine besonders effiziente Ausnutzung des Querschnitts des Tubus eines Trokars ermöglichen. Dabei ist vor allem das Verhältnis zwischen dem innerhalb des mittleren Schaftabschnitts zur Verfügung stehenden Bauraum und der mechanischen Steifigkeit des mittleren Schaftabschnitts einerseits und dem innerhalb des Lumens des Trokartubus neben dem mittleren Schaftabschnitt für ein oder mehrere weitere Instrumente verbleibenden Querschnitt besonders günstig.

Bei einem Endoskop, wie es hier beschrieben ist, ist der proximale Schaftabschnitt insbesondere zur Einführung in das Lumen eines Tubus vorgesehen.

Bei einem Endoskop, wie es hier beschrieben ist, ist der Rand des Querschnitts des proximalen Schaftabschnitts insbesondere kreisförmig.

Bei einem Endoskop, wie es hier beschrieben ist, entspricht der Querschnitt des proximalen Schaftabschnitts insbesondere dem Querschnitt des distalen Schaftabschnitts.

Der proximale Schaftabschnitt kann das Lumen eines Trokartubus, in den der Endoskopschaft vollständig eingeführt ist, teilweise oder vollständig verschließen. Wenn die Querschnitte des proximalen Schaftabschnitts und des distalen Schaftabschnitts einander entsprechen, können der proximale Schaftabschnitt und der distale Schaftabschnitt für eine Verwendung mit einem vorbestimmten Trokartubus vorgesehen, ausgebildet und insbesondere optimiert sein. Der Querschnitt des proximalen Schaftabschnitts und der Querschnitt des distalen Schaftabschnitts sind jeweils insbesondere so gewählt, dass der distale Schaftabschnitts reibungsarm durch das Lumen eines vorbestimmten Trokartubus, beispielsweise eines Standard-Trokartubus, hindurchgeführt und der proximale Schaftabschnitt spiel- und reibungsarm in das Lumen des gleichen Trokartubus eingesetzt werden kann.

Bei einem Endoskop, wie es hier beschrieben ist, ist insbesondere bezogen auf eine Projektion in Richtung parallel zur Längsachse des mittleren Schaftabschnitts der Querschnitt des mittleren Schaftabschnitts am Rand des Querschnitts des proximalen Schaftabschnitts angeordnet.

Wenn der proximale Schaftabschnitt in der vorgesehenen Weise in das Lumen eines vorbestimmten Trokartubus eingesetzt und darin insbesondere eine formschlüssig bis auf Spiel definierte Position einnimmt, liegt der mittlere Schaftabschnitts an der Innenwand des Trokartubus an oder ist nahe an dieser und parallel zu dieser angeordnet. Dadurch kann ein möglichst großer Teil des Lumens des Trokartubus für ein oder mehrere andere Instrumente verbleiben.

Bei einem Endoskop, wie es hier beschrieben ist, ist der Querschnitt des distalen Schaftabschnitts insbesondere gegenüber dem Querschnitt des mittleren Schaftabschnitts in einer ersten Richtung seitlich versetzt, wobei der Querschnitt des proximalen Schaftabschnitts gegenüber dem Querschnitt des mittleren Schaftabschnitts in einer zweiten Richtung seitlich versetzt ist, wobei die erste Richtung und die zweite Richtung einander entgegengesetzt oder voneinander verschieden sind.

Bei einem Endoskop, wie es hier beschrieben ist, stehen insbesondere bezogen auf eine Projektion in Richtung parallel zur Längsachse des mittleren Schaftabschnitts der Querschnitt des proximalen Schaftabschnitts und der Querschnitt des distalen Schaftabschnitts gegenüber dem Querschnitt des mittleren Schaftabschnitts in verschiedene Richtungen über.

Bei einem Endoskop, wie es hier beschrieben ist, stehen insbesondere bezogen auf eine Projektion in Richtung parallel zur Längsachse des mittleren Schaftabschnitts der Querschnitt des proximalen Schaftabschnitts und der Querschnitt des distalen Schaftabschnitts gegenüber dem Querschnitt des mittleren Schaftabschnitts in entgegengesetzten Richtungen über.

Der Querschnitt des proximalen Schaftabschnitts und der Querschnitt des distalen Schaftabschnitts stehen gegenüber dem Querschnitt des mittleren Schaftabschnitts insbesondere in entgegengesetzte Richtungen über. Im Fall eines geraden Endoskopschafts kann ein an dem mittleren Schaftabschnitt vorbeigeführtes gerades Instrument auch an dem distalen Schaftabschnitt vorbeigeführt werden.

Bei einem Endoskop, wie es hier beschrieben ist, geht der mittlere Schaftabschnitt insbesondere an einer Seite glatt in den distalen Schaftabschnitt über und an einer entgegengesetzten Seite glatt in den proximalen Schaftabschnitt über.

Ein glatter Übergang an einer Seite ist insbesondere ein stufenloser Übergang, bei dem die Oberflächen beider angrenzender Schaftabschnitte an dieser Seite fluchten.

Bei einem Endoskop, wie es hier beschrieben ist, ist bezogen auf eine Projektion in Richtung parallel zur Längsachse des mittleren Schaftabschnitts der Querschnitt des mittleren Schaftabschnitts insbesondere an einem Abschnitt des Rands des Querschnitts des proximalen Schaftabschnitts und an einem gegenüberliegenden Abschnitt des Rands des Querschnitts des distalen Schaftabschnitts angeordnet.

Bei einem Endoskop, wie es hier beschrieben ist, geht am Übergang zwischen dem distalen Schaftabschnitt und dem mittleren Schaftabschnitt der Querschnitt des distalen Schaftabschnitts insbesondere kontinuierlich in den Querschnitt des mittleren Schaftabschnitts über, wobei am Übergang zwischen dem mittleren Schaftabschnitt und dem proximalen Schaftabschnitt der Querschnitt des mittleren Schaftabschnitts kontinuierlich in den Querschnitt des proximalen Schaftabschnitts übergeht.

Ein kontinuierlicher Übergang von einem Querschnitt zu einem anderen Querschnitt ist insbesondere ein Übergang ohne oder im Wesentlichen ohne Stufe. Der Übergang ist insbesondere so ausgebildet, dass weder beim Einführen in einen Trokartubus noch beim Herausziehen aus einem Trokartubus der Übergang an einem Rand des Trokartubus verhaken oder formschlüssig gehemmt werden kann.

Ein Endoskop, wie es hier beschrieben ist, umfasst insbesondere ferner einen Arbeitskanal zur Aufnahme eines Instruments in dem proximalen Schaftabschnitt, wobei eine distale Austrittsöffnung des Arbeitskanals neben dem proximalen Ende des mittleren Schaftabschnitts angeordnet ist.

Der Arbeitskanal kann ein Hindurchführen eines Instruments durch den proximalen Schaftabschnitt ermöglichen. Ein durch den Arbeitskanal hindurchgeführtes und aus der distalen Austrittsöffhung des Arbeitskanals austretendes Instrument kann am mittleren Schaftabschnitt vorbei bis zu dem distalen Schaftabschnitt und optional darüber hinaus reichen. Das Endoskop kann mehrere Arbeitskanäle zur Aufnahme je eines oder mehrerer Instrumente in dem proximalen Schaftabschnitt aufweisen.

Bei einem Endoskop, wie es hier beschrieben ist, ist die Längsachse des distalen Endes des Arbeitskanals insbesondere parallel zu der Längsachse des proximalen Endes des mittleren Schaftabschnitts.

Bei einem Endoskop, wie es hier beschrieben ist, ist die Längsachse des Arbeitskanals insbesondere parallel zu der Längsachse des mittleren Schaftabschnitts.

Ein Endoskop, wie es hier beschrieben ist, umfasst insbesondere ferner eine Dichteinrichtung zum fluiddichten Verschließen des Arbeitskanals oder zum Reduzieren des Querschnitts des Arbeitskanals, wenn kein Instrument in dem Arbeitskanal angeordnet ist.

Die Dichteinrichtung kann einen Austritt von Insufflationsgas durch den Arbeitskanal hindurch verhindern oder vermindern.

Bei einem Endoskop, wie es hier beschrieben ist, ist der Querschnitt des Arbeitskanals in dem proximalen Schaftabschnitt insbesondere nicht kreisförmig.

Bei einem Endoskop, wie es hier beschrieben ist, füllt der Querschnitt des Arbeitskanals den Querschnitt des proximalen Schaftabschnitts abzüglich des Querschnitts des mittleren Schaftabschnitts insbesondere weitgehend aus.

Der Querschnitt des Arbeitskanals füllt den Querschnitt des proximalen Schaftabschnitts abzüglich des Querschnitts des mittleren Schaftabschnitts insbesondere dann weitgehend aus, wenn die Summe der Fläche des Querschnitts des Arbeitskanals und der Fläche des Querschnitts des mittleren Schaftabschnitts mindestens die Hälfte oder mindestens zwei Drittel oder mindestens drei Viertel oder mindestens vier Fünftel oder mindestens neun Zehntel der Fläche des Querschnitts des proximalen Schaftabschnitts beträgt.

Ein Endoskop, wie es hier beschrieben ist, umfasst insbesondere ferner eine Führungseinrichtung zum Führen eines Instruments an dem mittleren Schaftabschnitt.

Die Führungseinrichtung kann ferner zum Führen eines Instruments an dem distalen Schaftabschnitt vorgesehen und ausgebildet sein. Die Führungseinrichtung umfasst beispielsweise eine oder mehrere Längsnuten oder Längsstege zum formschlüssigen Führen eines Instruments. Alternativ oder zusätzlich kann die Führungseinrichtung einen oder mehrere paramagnetische oder ferromagnetische Bereiche zum magnetischen Führen eines Instruments an dem mittleren Schaftabschnitt und/oder an dem distalen Schaftabschnitt umfassen.

Die Führungseinrichtung kann formschlüssig, magnetisch oder auf andere Weise eine Position eines Instruments relativ zu dem Endoskopschaft in einer oder zwei Richtungen orthogonal zu der Längsachse des Endoskopschafts definieren. Alternativ oder zusätzlich kann die Führungseinrichtung eine Position eines Instruments in Richtung parallel zu der Längsachse des Endoskopschafts relativ zu dem Endoskopschaft definieren, beispielsweise durch einen mechanischen Anschlag oder mittels einer Rasteinrichtung oder auf magnetische Weise.

Ein Reinigungsinstrument zur Reinigung oder Trocknung einer Lichteintrittsfläche an einem distalen Schaftabschnitt eines Endoskops umfasst einen Instrumentenschaft und ein Werkzeug am distalen Ende des Instrumentenschafts zum Reinigen oder Trocknen der Lichteintrittsfläche am distalen Schaftabschnitt des Endoskops, wobei der Instrumentenschaft zur Anordnung neben einem Endoskopschaft des Endoskops vorgesehen und ausgebildet ist.

Ein Reinigungsinstrument zur Reinigung oder Trocknung der Lichteintrittsfläche an dem distalen Schaftabschnitt eines Endoskops, wie es hier beschrieben ist, umfasst einen Instrumentenschaft und ein Werkzeug am distalen Ende des Instrumentenschafts zum Reinigen oder Trocknen der Lichteintrittsfläche an dem distalen Schaftabschnitt des Endoskops, wobei der Instrumentenschaft zur Anordnung neben dem Endoskopschaft des Endoskops vorgesehen und ausgebildet ist.

Der Instrumentenschaft ist insbesondere länger als der Endoskopschaft des Endoskops, für das das Reinigungsinstrument vorgesehen ist. Der Instrumentenschaft ist insbesondere nicht zur Anordnung in einem Arbeitskanal, der sich entlang eines gesamten Endoskopschafts bis zu dem distalen Ende des Endoskops erstreckt, vorgesehen und ausgebildet. Der Instrumentenschaft ist insbesondere nicht vorgesehen, um einen Endoskopschaft aufzunehmen. Der Instrumentenschaft ist insbesondere zur Anordnung neben dem mittleren Schaftabschnitt und optional auch neben dem distalen Schaftabschnitt eines Endoskops vorgesehen und ausgebildet.

Bei einem Reinigungsinstrument, wie es hier beschrieben ist, ist der Querschnitt des Instrumentenschafts insbesondere so an den Querschnitt des mittleren Schaftabschnitts des Endoskops angepasst, dass der Gesamtquerschnitt einer parallelen Anordnung des Instrumentenschafts und des mittleren Schaftabschnitts des Endoskops nicht größer ist als der Querschnitt des distalen Schaftabschnitts des Endoskops.

Die parallele Anordnung des Instrumentenschafts und des mittleren Schaftabschnitts des Endoskops ist eine Anordnung, bei der die Längsachse des Instrumentenschafts parallel zur Längsachse des mittleren Schaftabschnitts des Endoskops ist. Die parallele Anordnung des Instrumentenschafts und des mittleren Schaftabschnitts ist insbesondere eine Anordnung, bei der der Instrumentenschaft an dem mittleren Schaftabschnitt des Endoskops anliegt.

Der Gesamtquerschnitt der parallelen Anordnung ist insbesondere kleiner als der Querschnitt des distalen Schaftabschnitts des Endoskops. Wenn das Endoskop proximal des mittleren Schaftabschnitts einen proximalen Schaftabschnitt mit einem Querschnitt, der größer ist als der Querschnitt des mittleren Schaftabschnitts, aufweist, ist der Gesamtquerschnitt der parallelen Anordnung des Instrumentenschafts und des mittleren Schaftabschnitts des Endoskops insbesondere auch kleiner oder nicht größer als der Querschnitt des proximalen Schaftabschnitts des Endoskops.

Ein Reinigungsinstrument, wie es hier beschrieben ist, umfasst insbesondere ferner einen Fluidkanal in dem Instrumentenschaft, wobei das Werkzeug eine Düse, die mit dem distalen Ende des Fluidkanals verbunden ist, umfasst, wobei die Düse zum Leiten eines Fluids zu der Lichteintrittsfläche des Endoskops vorgesehen und ausgebildet ist.

Der Fluidkanal und die Düse können eine Erzeugung einer Strömung, insbesondere eines scharfen Strahls, von Insufflationsgas zum Trocknen der Lichteintrittsfläche oder zum Abtragen einer flüssigen oder festen Ablagerung an der Lichteintrittsfläche ermöglichen. Alternativ oder zusätzlich können der Fluidkanal und die Düse eine Erzeugung eines auf die Lichteintrittsfläche gerichteten Strahls aus einer Kochsalzlösung oder einer anderen Spülflüssigkeit zum Abtragen einer flüssigen oder festen Ablagerung an der Lichteintrittsfläche ermöglichen.

Bei einem Reinigungsinstrument, wie es hier beschrieben ist, ist die Düse insbesondere vorgesehen und ausgebildet zur Ausbildung eines Fluidstroms an der Lichteintrittsfläche des Endoskops zumindest entweder zur Entfernung einer Ablagerung an der Lichteintrittsfläche oder zur Verhinderung der Entstehung einer Ablagerung an der Lichteintrittsfläche.

Ein kontinuierlicher, eine ausreichende Strömungsgeschwindigkeit aufweisender Gasstrom kann geeignet sein, Partikel oder Tropfen einer Flüssigkeit mitzureißen und wegzutragen, bevor sie die Lichteintrittsfläche erreichen können.

Ein Reinigungsinstrument, wie es hier beschrieben ist, umfasst insbesondere ferner eine Führungseinrichtung zum Führen des Reinigungsinstruments an einen Schaft des Endoskops.

Die Führungseinrichtung umfasst insbesondere eine Längsnut, einen Längssteg, einen paramagnetischen oder ferromagnetischen Bereich zum formschlüssigen oder magnetischen Führen des Reinigungsinstruments an einem Endoskopschaft des Endoskops. Die Führungseinrichtung ist insbesondere zur Wechselwirkung mit einer korrespondierenden Führungseinrichtung an dem Endoskopschaft vorgesehen und ausgebildet. Die Führungseinrichtung kann punktförmig oder linienförmig sein und sich entlang des gesamten Instrumentenschafts erstrecken oder nur an dem distalen Ende des Instrumentenschafts oder an dem Werkzeug angeordnet sein.

Äußere Einwirkung, der Rückstoß eines austretenden Fluids oder die unmittelbare mechanische Wechselwirkung des Werkzeugs mit der Lichteintrittsfläche oder mit Ablagerungen auf der Lichteintrittsfläche kann eine Kraft erzeugen, die eine Verformung des Reinigungsinstruments und eine Bewegung des Werkzeugs weg von seiner vorgesehenen Position bewirkt. Die Führungseinrichtung kann trotz des Wirkens einer solchen Kraft eine definierte Position des Werkzeugs relativ zu dem Endoskopschaft ermöglichen.

Bei einem Reinigungsinstrument, wie es hier beschrieben ist, umfasst das Werkzeug insbesondere eine Wischeinrichtung zum Abstreifen von Ablagerungen an der Lichteintrittsfläche des Endoskops.

Die Wischeinrichtung umfasst insbesondere ein elastisches Wischerblatt oder einen Rakel aus Silikon, Gummi oder einem anderen elastischen Material zum Abstreifen von Schmutz, Feuchtigkeit oder anderen festen oder flüssigen Ablagerungen von der Lichteintrittsfläche des Endoskops. Die Wischeinrichtung kann vorgesehen und ausgebildet sein, um durch eine Rotationsbewegung des Instrumentenschafts und mit ihm des Werkzeugs, durch eine Bewegung des Instrumentenschafts parallel zu seiner Längsachse oder auf andere Weise relativ zu der Lichteintrittsfläche des Endoskops bewegt zu werden.

Bei einem Reinigungsinstrument, wie es hier beschrieben ist, weist das Werkzeug insbesondere ein Gelenk auf, das ein Anlegen einer Wischlippe an die Lichteintrittsfläche des Endoskops ermöglicht.

Das Gelenk kann ein Festkörpergelenk sein. Zusätzlich zu dem Gelenk kann eine elastische Einrichtung vorgesehen sein, die die Wischlippe an die Lichteintrittsfläche des Endoskops andrücken oder anlegen kann.

Das Gelenk kann eine Bewegung des Werkzeugs zwischen einer Einführposition und einer Arbeitsposition ermöglichen. In der Einführposition des Werkzeugs kann das Reinigungsinstrument durch einen Arbeitskanal in einem Endoskop hindurch und neben einem mittleren Schaftabschnitt des Endoskops durch das Lumen eines Trokartubus hindurchgeführt werden. In der Arbeitsposition kann eine Lichteintrittsfläche des Endoskops gereinigt werden. Das Gelenk kann ferner im Fall einer geneigten oder gekrümmten Lichteintrittsfläche ermöglichen, dass das Werkzeugs der Lichteintrittsfläche folgt.

Bei einem Reinigungsinstrument, wie es hier beschrieben ist, umfasst das Werkzeug insbesondere eine Wischlippe, die nicht-parallel zur Längsachse des Instrumentenschafts angeordnet und vorgesehen ist, um bei einer Bewegung des Reinigungsinstruments in Richtung parallel zur Längsachse des Instrumentenschafts relativ zu dem Endoskop Ablagerungen von der Lichteintrittsfläche des Endoskops abzustreifen.

Die Wischlippe ist insbesondere orthogonal oder im Wesentlichen orthogonal zur Längsachse des Instrumentenschafts angeordnet. Die Wischlippe ist im Wesentlichen orthogonal zur

Längsachse des Instrumentenschafts angeordnet, wenn der Winkel zwischen der Wischlippe und der Längsachse des Instrumentenschafts mindestens 60 Grad oder mindestens 70 Grad oder mindestens 80 Grad beträgt. Insbesondere in Kombination mit einem Gelenk, das ein Schwenken des Werkzeugs oder zumindest der Wischlippe um eine Schwenkachse orthogonal oder im Wesentlichen orthogonal zur Längsachse des Instrumentenschafts ermöglicht, kann die Wischlippe durch bloße Bewegung des Instrumentenschafts parallel zu seiner Längsachse über die Lichteintrittsfläche geführt werden.

Bei einem Reinigungsinstrument, wie es hier beschrieben ist, ist die Wischlippe insbesondere um eine Schwenkachse schwenkbar, um Ablagerungen von der Lichteintrittsfläche des Endoskops abzustreifen.

Die Schwenkachse ist insbesondere parallel zu der Längsachse des Instrumentenschafts. Die Schwenkbewegung der Wischlippe kann durch eine Antriebswelle in dem Instrumentenschaft oder durch Rotation des Instrumentenschafts um seine Längsachse erzeugt werden. Die Wischlippe kann in diesem Fall die Lichteintrittsfläche auf ähnliche Weise reinigen wie ein Wischerblatt eine Windschutzscheibe eines Fahrzeugs.

Ein Endoskopiesystem umfasst ein Endoskop, wie es hier beschrieben ist, und ein Reinigungsinstrument, wie es hier beschrieben ist.

Ein Endoskopiesystem umfasst ein Endoskop, wie es hier beschrieben ist, und einen Trokartubus mit einem Lumen zur Aufnahme des Endoskopschafts des Endoskops, wobei der Endoskopschaft des Endoskops und der Trokartubus so ausgebildet sind, das der distale Schaftabschnitt des Endoskops vollständig durch den Trokartubus hindurchgeführt werden kann.

Insbesondere sind der Querschnitt des distalen Schaftabschnitts des Endoskopschafts und der Querschnitt des Lumens des Trokartubus so ausgebildet, dass der distale Schaftabschnitts des Endoskops reibungsarm vollständig durch den Trokartubus hindurchgeführt werden kann.

Der Trokartubus kann gerade oder gekrümmt sein. Wenn das Endoskop einen gekrümmten Schaft aufweist, weist der Trokartubus insbesondere eine entsprechende Krümmung auf.

Bei einem Endoskopiesystem, wie es hier beschrieben ist, ist der Trokartubus oder ein zylindrischer Abschnitt des Lumens des Trokartubus insbesondere nicht länger als der mittlere Schaftabschnitt des Endoskops.

Der Trokartubus oder ein zylindrischer Abschnitt des Lumens des Trokartubus ist insbesondere kürzer als der mittlere Schaftabschnitt des Endoskops. Wenn der Trokartubus oder ein zylindrischer Abschnitt des Lumens des Trokartubus nicht länger als der mittlere Schaftabschnitt des Endoskops ist, kann der Endoskopschaft immer dann, wenn nur der mittlere Schaftabschnitt in dem Trokartubus oder in dem zylindrischen Abschnitt des Lumens des Trokartubus angeordnet ist, in Richtung orthogonal zu der Längsachse des mittleren Schaftabschnitts bewegt werden. Diese Bewegung kann zwischen dem Hindurchführen des distalen Schaftabschnitts durch den Trokartubus oder den zylindrischen Abschnitt des Lumens des Trokartubus und dem Einführen eines proximalen Schaftabschnitts in den Trokartubus ausgeführt werden.

Bei einem Endoskopiesystem, wie es hier beschrieben ist, sind der proximale Schaftabschnitt des Endoskops und der Trokartubus dafür vorgesehen und ausgebildet, dass der Trokartubus den proximalen Schaftabschnitt aufnimmt.

Insbesondere sind der proximale Schaftabschnitt des Endoskops und der Trokartubus so ausgebildet, dass der proximale Schaftabschnitt des Endoskops in dem Trokartubus spiel- und reibungsarm geführt ist.

Ein Endoskopiesystem, wie es hier beschrieben ist, umfasst insbesondere ferner ein Reinigungsinstrument, wie es hier beschrieben ist.

Ein Endoskopiesystem, wie es hier beschrieben ist, umfasst insbesondere ferner ein Reinigungsinstrument, wie es hier beschrieben ist, wobei der mittlere Schaftabschnitt des Endoskops, der Instrumentenschaft des Reinigungsinstruments und der Trokartubus dafür vorgesehen und ausgebildet sind, dass der Querschnitt des mittleren Schaftabschnitts des Endoskops und der Querschnitt des Instrumentenschafts des Reinigungsinstruments den Querschnitt des Lumens des Trokartubus weitgehend ausfüllen.

Der Querschnitt des mittleren Schaftabschnitts des Endoskops und der Querschnitt des Instrumentenschafts des Reinigungsinstruments füllen den Querschnitt des Lumens des Trokartubus dann weitgehend aus, wenn der Gesamtquerschnitt des mittleren Schaftabschnitts des Endoskops und des Instrumentenschafts des Reinigungsinstruments mindestens die Hälfte oder mindestens zwei Drittel oder mindestens drei Viertel oder mindestens vier Fünftel oder mindestens neun Zehntel der Fläche des Querschnitts des Lumens des Trokartubus einnehmen.

Bei einem Verfahren zur Vorbereitung der Verwendung eines Endoskops werden ein Endoskop in einen Trokartubus bis zu einer vorbestimmten Position eingeführt, das Endoskop in dem Trokartubus in einer Richtung orthogonal zu der Einführrichtung bewegt und das Endoskop bis zu einer Arbeitsposition in dem Trokartubus bewegt.

Das Verfahren ist insbesondere mit einem Endoskop und einem Trokartubus, wie sie hier beschrieben sind oder mit einem Endoskop und einem Trokartubus mit Merkmalen, Eigenschaften und Funktionen, wie sie hier beschrieben sind, ausführbar. Insbesondere ist das Verfahren mit einem Endoskop ausführbar, das einen mittleren Schaftabschnitt, dessen Querschnitt kleiner ist als der Querschnitt eines distalen Schaftabschnitts, aufweist. Die vorbestimmte Position ist insbesondere eine Position, in der lediglich der mittlere Schaftabschnitt in dem Trokartubus oder in einem zylindrischen Abschnitt des Lumens des Trokartubus angeordnet ist. In der Arbeitsposition des Endoskops relativ zu dem Trokartubus ist insbesondere ein proximaler Schaftabschnitt des Endoskops in den Trokartubus eingeführt.

Bei einem Verfahren, wie es hier beschrieben ist, kann ein Reinigungsinstrument oder ein anderes Instrument an einem Schaftabschnitt (insbesondere an einem mittleren Schaftabschnitt) des Endoskops in der Arbeitsposition vorbeigeführt werden.

Bei einem optionalen vorangehenden Schritt kann das Instrument durch einen Arbeitskanal des Endoskops hindurchgeführt werden. Die distale Austrittsöffnung des Arbeitskanals ist insbesondere neben dem proximalen Ende des Schaftabschnitts, der bei der vorbestimmten Position in dem Trokartubus angeordnet ist.

Bei einem optionalen weiteren Schritt wird eine Lichteintrittsfläche des Endoskops mit dem Instrument gereinigt.

### Kurzbeschreibung der Figuren

Nachfolgend werden Ausführungsformen anhand der beigefügten Figuren näher erläutert. Es zeigen:
- Figur 1: eine schematische Darstellung eines Endoskops gemäß der Erfindung;

- Figur 2: eine schematische Darstellung eines Reinigungsinstruments;
- Figur 3: eine schematische Darstellung eines Trokartubus;
- Figur 4: eine schematische Darstellung eines Endoskopiesystems aus dem Endoskop aus Figur 1 und dem Trokartubus aus Figur 3;
- Figur 5: eine weitere schematische Darstellung des Endoskopiesystems aus Figur 4;
- Figur 6: eine weitere schematische Darstellung des Endoskopiesystems aus den Figuren 4 und 5;

- Figur 7: eine weitere schematische Darstellung des Endoskopiesystems aus den Figuren 4 bis 6;
- Figur 8: eine schematische Darstellung eines Endoskopiesystems aus dem Endoskop aus Figur 1, dem Reinigungsinstrument aus Figur 2 und dem Trokartubus aus Figur 3;
- Figur 9: eine weitere schematische Darstellung des Endoskopiesystems aus Figur 8;
- Figur 10: eine schematische Darstellung eines weiteren Reinigungsinstruments;

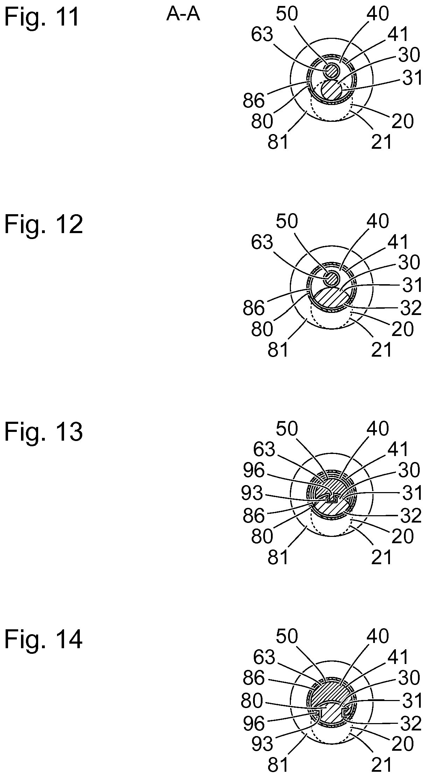

- Figur 11: eine schematische Darstellung eines Querschnitts des Endoskopiesystems aus den Figuren 8 und 9;
- Figur 12: eine schematische Darstellung eines Querschnitts durch ein weiteres Endoskopiesystem;
- Figur 13: eine schematische Darstellung eines Querschnitts durch ein weiteres Endoskopiesystem;
- Figur 14: eine schematische Darstellung eines Querschnitts durch ein weiteres Endoskopiesystem;

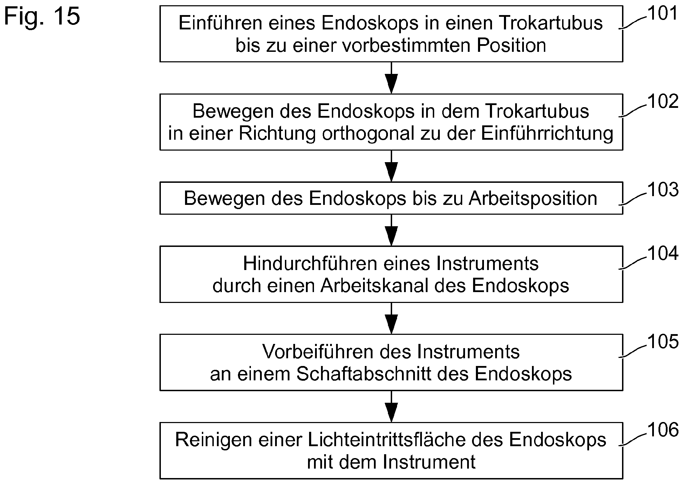

- Figur 15: ein schematisches Flussdiagramm eines Verfahrens zur Vorbereitung einer Verwendung eines Endoskopiesystems.

### Beschreibung der Ausführungsformen

Figur 1 zeigt eine schematische Darstellung eines Endoskops 10 mit einer Lichteintrittsfläche 11 am distalen Ende 12 des Endoskops 10, einem Endoskopschaft 13 und einer Handhabungseinrichtung 14, die das proximale Ende 15 des Endoskops 10 bildet. Der Endoskopschaft 13 weist mehrere zylindrische oder im Wesentlichen zylindrische Schaftabschnitte, 20, 30, 40 auf. Jeder der Schaftabschnitte 20, 30, 40 kann einen kreisförmigen oder einen anderen Querschnitt aufweisen. Bei dem dargestellten Beispiel weist jeder Schaftabschnitt 20, 30, 40 einen kreisförmigen Querschnitt auf, die Schaftabschnitt 20, 30, 40 weisen also die Form von Kreiszylindern auf.

Der äußere Rand bzw. die Kontur des Querschnitts des distalen Schaftabschnitts 20 ist kongruent oder im Wesentlichen kongruent zu dem äußeren Rand des Querschnitts des proximalen Schaftabschnitts 40, d. h. der distale Schaftabschnitt 20 und der proximale Schaftabschnitt 40 weisen den gleichen Durchmesser bzw. den gleichen Radius auf. Der mittlere Schaftabschnitt 30 weist einen deutlich kleineren Querschnitt mit einem deutlich kleineren Durchmesser auf.

In dem distalen Schaftabschnitt 20 kann eine Kamera bzw. eine Kombination aus einem Objektiv und einem Bildsensor angeordnet sein. Der Bildsensor wandelt ein von dem Objektiv erzeugtes Bild in ein analoges oder digitales Signal, das mittels einer oder mehrerer Signalleitungen in dem Endoskopschaft 13 zu dem proximalen Ende 15 des Endoskops 10 übertragen wird. Alternativ kann der distale Schaftabschnitt 20 ein Objektiv zum Erfassen eines realen Zwischenbilds, das mittels eines Relaislinsensystems in dem Endoskopschaft 13 zu dem proximalen Ende des Endoskops 10 übertragen wird, aufweisen.

In dem distalen Schaftabschnitt 20 kann ferner eine Vorrichtung zum Verändern der Blickrichtung des Endoskops angeordnet sein, beispielsweise ein schwenkbares Prisma, an dessen Oberfläche Totalreflexion stattfindet, oder andere schwenkbare reflektierende Flächen.

Neben der Lichteintrittsfläche 11 können ferner eine oder mehrere Lichtaustrittsflächen, durch die Beleuchtungslicht zur Beleuchtung eines zu betrachtenden Objekts austritt, angeordnet sein. Eine nachfolgend beschriebene Reinigung einer Lichteintrittsfläche kann auch die Reinigung einer oder mehrerer benachbarter Lichtaustrittsflächen beinhalten.

Der distale Schaftabschnitt 20 ist derart mit dem mittleren Schaftabschnitt 30 mechanisch starr verbunden, dass die Längsachse 28 des distalen Schaftabschnitts 20 parallel zu der Längsachse 38 des mittleren Schaftabschnitts 30 ist, und dass die Längsachse 28 des distalen Schaftabschnitts 20 gegenüber der Längsachse 38 des mittleren Schaftabschnitts 30 seitlich versetzt ist. In einer Projektion parallel zu den Längsachsen 28, 38 des distalen Schaftabschnitts 20 und des mittleren Schaftabschnitts 30 steht der distale Schaftabschnitt 20 in einer Richtung (Figur 1: unten) über, während an der gegenüberliegenden Seite (in Figur 1: oben) die Kontur des distalen Schaftabschnitts 20 mit der Kontur des mittleren Schaftabschnitts 30 fluchtet.

Zwischen dem distalen Schaftabschnitt 20 und dem mittleren Schaftabschnitt 30 ist bei dem dargestellten Beispiel ein konischer oder im Wesentlichen konischer oder kegelförmiger Übergangsbereich vorgesehen. Alternativ kann ein abrupter bzw. stufenförmiger Übergang zwischen dem distalen Schaftabschnitt 20 und dem mittleren Schaftabschnitt 30 vorgesehen sein.

Der proximale Schaftabschnitt 40 ist gegenüber dem mittleren Schaftabschnitt 30 seitlich versetzt. In einer Projektion parallel zu der Längsachse 38 des mittleren Schaftabschnitts 30 und zu der in Figur 1 nicht dargestellten Längsachse des proximalen Schaftabschnitts 40 geht die Kontur des mittleren Schaftabschnitts an einer Seite (in Figur 1: unten) glatt in die Kontur des proximalen Schaftabschnitts 40 über, während die Kontur des proximalen Schaftabschnitts an einer gegenüberliegenden Seite (in Figur 1: oben) gegenüber der Kontur des mittleren Schaftabschnitts 30 übersteht.

Bei dem dargestellten Beispiel ist der Übergang zwischen dem mittleren Schaftabschnitt 30 und dem proximalen Schaftabschnitt 40 stufenförmig. Der Querschnitt des Endoskopschafts 13 ändert sich also von distal (in Figur 1: links) nach proximal (in Figur 1: rechts) abrupt ohne Übergangsbereich. Alternativ kann ein kontinuierlicher bzw. glatter Übergang zwischen dem mittleren Schaftabschnitt 30 und dem proximalen Schaftabschnitt 40 vorgesehen sein.

In dem proximalen Schaftabschnitt 40 und in der Handhabungseinrichtung 14 des Endoskops 10 ist ein Arbeitskanal 50 vorgesehen, dessen Konturen in Figur 1 durch gestrichelte Linien angedeutet sind. Der Arbeitskanal 50 ist vorgesehen und ausgebildet, um ein Instrument, beispielsweise ein Reinigungsinstrument zum Reinigen der Lichteintrittfläche 11 an dem distalen Ende 12 des Endoskops 10 aufzunehmen.

In dem Arbeitskanal 50 ist eine Dichteinrichtung 52 angeordnet. Die Dichteinrichtung 52 ist vorgesehen und ausgebildet, um einen verbleibenden ringförmigen Spalt zwischen der inneren Oberfläche des Arbeitskanals 50 und einer äußeren Oberfläche eines in den Arbeitskanal 50 eingesetzten Instruments fluiddicht zu verschließen bzw. abzudichten. Alternativ oder zusätzlich ist die Dichteinrichtung 52 vorgesehen und ausgebildet, um den Arbeitskanal 50 fluiddicht zu verschließen oder zumindest seinen Querschnitt erheblich zu verringern, wenn kein Instrument in den Arbeitskanal 50 eingesetzt ist.

Eine distale Austrittsöffnung 53 des Arbeitskanals 50 ist neben dem proximalen Ende 34 des mittleren Schaftabschnitts 30 angeordnet. Die Längsachse 58 des Arbeitskanals 50 ist parallel zu der Längsachse 38 des mittleren Schaftabschnitts 30 und parallel zu der in Figur 1 nicht dargestellten Längsachse des proximalen Schaftabschnitts 40.

Figur 2 zeigt eine schematische Darstellung eines Reinigungsinstruments 60 zum Reinigen einer Lichteintrittsfläche eines Endoskops, insbesondere der Lichteintrittsfläche 11 des anhand der Figur 1 dargestellten Endoskops 10. Das Reinigungsinstrument 60 umfasst ein distales Ende 61, einen Instrumentenschaft 63 und eine Handhabungseinrichtung in Form eines Rads 64, das das proximale Ende des Reinigungsinstruments 60 bildet. Das Rad 64 ist insbesondere vorgesehen, um das Reinigungsinstrument um die Längsachse 68 des Instrumentenschafts 63 manuell zu rotieren.

Das distale Ende 61 des Reinigungsinstruments 60 wird durch ein Reinigungswerkzeug 70 mit einer Wischlippe 72 zum Abstreifen von flüssigen oder festen Ablagerungen an einer Lichteintrittsfläche eines Endoskops gebildet. Das Reinigungswerkzeug 70 ist durch ein Gelenk 76 mit dem Instrumentenschaft 63 gelenkig verbunden. Das Gelenk 76 ermöglicht ein Schwenken des Reinigungswerkzeugs 70 um eine Schwenkachse 78 orthogonal zu der Längsachse 68 des Instrumentenschafts 63 und orthogonal zu der Zeichenebene der Figur 2.

Das Reinigungswerkzeug 70 ist in Figur 2 in durchgezogenen Linien in einer Einführposition und in gestrichelten Linien in einer Arbeits- bzw. Reinigungsposition angedeutet. Das Reinigungsinstrument 60 umfasst eine elastische Einrichtung oder eine andere Einrichtung, die das Reinigungswerkzeug 70 in die in Figur 2 in gestrichelten Linien angedeutete Arbeits- bzw. Reinigungsposition bewegt. Gegen die elastische Kraft der elastischen Einrichtung kann das Reinigungswerkzeug 70 in die in Figur 2 in durchgezogenen Linien gezeigte Einführposition bewegt werde.

Figur 3 zeigt eine schematische Darstellung eines Trokartubus 80 mit einem proximalen Ende 81 und einem distalen Ende 82. Das proximale Ende 81 des Trokartubus 80 kann durch eine in Figur 3 angedeutete Handhabungseinrichtung gebildet werden. Ein Lumen 86 erstreckt sich von dem proximalen Ende 81 bis zu dem distalen Ende 82 des Trokartubus. Das Lumen 86 weist bei dem dargestellten Beispiel durchgehend von dem proximalen Ende 81 bis zu dem distalen Ende 82 die Gestalt eines Zylinders, insbesondere eines Kreiszylinders, auf. Die Konturen des Lumens 86 sind in Figur 3 nicht sichtbar und deshalb nur in gestrichelten Linien angedeutet.

Figur 4 zeigt eine schematische Darstellung eines Endoskopiesystem, das ein Endoskop 10 und einen Trokartubus 80, insbesondere das anhand der Figur 1 dargestellte Endoskop 10 und den anhand der Figur 3 dargestellten Trokartubus 80 umfasst. In Figur 4 sind nur der distale Schaftabschnitt 20 und ein Teil des mittleren Schaftabschnitts 30 des Endoskops 10 sichtbar. Der distale Schaftabschnitt 20 ist nahe dem proximalen Ende 81 des Trokartubus 80 angeordnet und relativ zu dem Lumen 86 in dem Trokartubus 80 so positioniert und orientiert, dass der distale Schaftabschnitt 20 mit einer Bewegung des gesamten Endoskops 10 relativ zu dem Trokartubus 80 nach distal (in Figur 4: links) durch das Lumen 86 des Trokartubus 80 hindurch bewegt werden kann.

Figur 5 zeigt eine weitere schematische Darstellung des Endoskopiesystems aus Figur 4. In Figur 5 ist eine Situation bzw. Konfiguration gezeigt, die entsteht, wenn das Endoskop 10 ausgehend von der in Figur 4 gezeigten Position relativ zu dem Trokartubus 80 parallel zu der Längsachse 88 des Trokartubus 80, parallel zu der Längsachse 28 des distalen Schaftabschnitts 20, parallel zu der Längsachse 38 des mittleren Schaftabschnitts 30 und parallel zu der Längsachse 48 des proximalen Schaftabschnitts 40 nach distal verschoben wird.

Bei der in Figur 5 dargestellten Position des Endoskops 10 relativ zu dem Trokartubus 80 liegt eine Seite (in Figur 5: die obere Seite) des mittleren Schaftabschnitts 30 an der inneren Oberfläche des Lumens 86 des Trokartubus 80 an oder weist einen geringen Abstand von dieser auf.

Figur 6 zeigt eine weitere schematische Darstellung des Endoskopiesystems aus den Figuren 4 und 5. Die in Figur 6 gezeigte Situation entsteht, wenn das Endoskop 10 ausgehend von der in Figur 5 gezeigten Position relativ zu dem Trokartubus in einer Richtung orthogonal zu der Längsachse 28 des distalen Schaftabschnitts 20, orthogonal zu der Längsachse des mittleren Schaftabschnitts und orthogonal zu der Längsachse 88 des Trokartubus 80 bewegt wird, und zwar bezogen auf die Darstellung in Figur 6 von oben nach unten.

Bei der in Figur 6 dargestellten Position des Endoskop 10 relativ zu dem Trokartubus 80 liegt der mittlere Schaftabschnitt 30 an einer in Vergleich zu der in Figur 5 gezeigten Situation entgegengesetzten Seite (in Figur 6: unten) an der inneren Oberfläche des Lumens 86 des Trokartubus 80 an oder weist einen geringen Abstand von dieser auf.

Figur 7 zeigt weitere schematische Darstellung des Endoskopiesystems aus den Figuren 4 bis 6. Die in Figur 7 gezeigte Situation entsteht, wenn das Endoskop 10 ausgehend von der in Figur 6 gezeigten Position relativ zu dem Trokartubus 80 in Richtung parallel zu der Längsachse 28 des distalen Schaftabschnitts 20, parallel zu der Längsachse 38 des mittleren Schaftabschnitts 30 und parallel zu der Längsachse des Trokartubus 80 weiter nach distal bewegt wird. Dabei wird der proximale Schaftabschnitt 40 in das Lumen 86 des Trokartubus 80 eingeführt.

Bei der in Figur 7 gezeigten Situation ist der proximale Schaftabschnitt 40 vollständig in einen proximalen Bereich des Lumens 86 des Trokartubus 80 eingeführt. Die Handhabungseinrichtung 14 liegt an dem proximalen Ende 81 des Trokartubus 80 an.

Figur 8 zeigt eine schematische Darstellung eines Endoskopiesystems, das ähnlich wie das anhand der Figuren 4 bis 7 dargestellte Endoskopiesystem ein Endoskop 10 und einen Trokartubus 80, insbesondere das anhand der Figur 1 dargestellte Endoskop 10 und den anhand der Figur 3 dargestellten Trokartubus 80 umfasst. Ferner umfasst das in Figur 8 gezeigte Endoskopiesystem ein Reinigungsinstrument 60, insbesondere das anhand der Figur 2 dargestellte Reinigungsinstrument 60.

Bei der in Figur 8 dargestellten Situation nimmt das Reinigungswerkzeug 70 an dem distalen Ende des Reinigungsinstruments 60 die in Figur 2 in durchgezogenen Linien gezeigten Einführposition relativ zu dem Instrumentenschaft 63 ein. Das Reinigungswerkzeug 70 ist von proximal her vollständig durch den Arbeitskanal 50 in dem Endoskop 10 in das Lumen 86 in dem Trokartubus 80 eingeführt. Der Instrumentenschaft 63 des Reinigungsinstruments 60 ist teilweise in den Arbeitskanal 50 des Endoskops 50 und teilweise das Lumen 86 des Trokartubus 80 eingeführt.

Figur 9 zeigt eine weitere schematische Darstellung des Endoskopiesystems aus Figur 8. Die in Figur 9 dargestellte Situation entsteht, wenn das Reinigungsinstrument 60 von der in Figur 8 gezeigten Position relativ zu dem Endoskop 10 und dem Trokartubus 80 parallel zu der Längsachse 68 des Instrumentenschafts 63 und parallel zu der Längsachse 58 des Arbeitskanals 50 nach distal bis zu der in Figur 9 gezeigten Arbeits- bzw. Reinigungsposition bewegt wird.

Die bei Figur 2 beschriebene elastische Rückstellkraft einer in den Figuren nicht gezeigten elastischen Einrichtung bewegt das Reinigungswerkzeug in die in Figur 9 gezeigte Reinigungsposition, in der die Wischlippe 72 an der Lichteintrittsfläche 11 des Endoskops 10 anliegt. Das Rad 64 kann manuell um die Längsachse 68 des Instrumentenschafts 63 rotiert werden. Dabei rotiert der Instrumentenschaft 63 zusammen mit dem Werkzeug 70 um die Längsachse 68 des Instrumentenschafts 63, und die Wischlippe 72 des Reinigungswerkzeugs 70 wird über die Lichteintrittsfläche 11 des Endoskops 10 bewegt. Dadurch können feste und flüssige Ablagerungen an der Lichteintrittsfläche 11 des Endoskops 10 und optional an benachbarten Lichtaustrittsflächen abgestreift bzw. zur Seite geschoben werden, um eine Erfassung eines scharfen, kontrastreichen und hellen Bilds zu ermöglichen.

Figur 10 zeigt eine schematische Darstellung eines weiteren Reinigungsinstruments 60, das in einigen Merkmalen, Eigenschaften und Funktionen dem anhand der Figuren 2, 8 und 9 dargestellten Reinigungsinstrument ähnelt. Nachfolgend sind insbesondere Merkmale, Eigenschaften und Funktionen des in Figur 10 gezeigten Reinigungsinstruments 60 beschrieben, in denen dieses sich von dem anhand der Figuren 2, 8 und 9 dargestellten Reinigungsinstrument unterscheidet. Im Unterschied zu den Darstellungen in den Figuren 1 bis 9 ist das Reinigungsinstrument 60 in Figur 10 in einem Längsschnitt gezeigt. In gestrichelten Linien sind ein Endoskop 10 und ein Trokartubus 80, insbesondere das anhand der Figur 1 dargestellte Endoskops 10 und der anhand der Figur 3 dargestellte Trokartubus angedeutet. Das Reinigungsinstrument 60 kann zusammen mit dem Endoskop 10 und/oder mit dem Trokartubus 80 ein Endoskopiesystem bilden. Die in Figur 10 angedeutete Position des Reinigungsinstruments 60 ist die vorgesehene Arbeits- bzw. Reinigungsposition relativ zu dem Endoskop 10.

Das Reinigungsinstrument 60 weist einen Fluidkanal 73 in dem Instrumentenschaft 63 auf, der mit einer Düse 74 an dem distalen Ende des Reinigungsinstruments 60 verbunden ist. Ein Gas, eine Kochsalzlösung oder eine andere Reinigungsflüssigkeit oder ein anderes Reinigungsfluid kann durch den Fluidkanal 73 zu der Düse 74 gefördert werden, durch die Düse 74 austreten und einen Fluidstrom 75 bilden. Die Düse 74 ist so angeordnet und ausgebildet, dass der Fluidstrom 75 auf die Lichteintrittsfläche 11 trifft und feste oder flüssige Ablagerungen an der Lichteintrittsfläche 11 abträgt. Alternativ oder zusätzlich kann die Düse 74 so ausgebildet sein, dass ein von der Düse 74 geformter Gasstrom 75 entlang der Lichteintrittsfläche 11 des Endoskops 10 die Bildung von festen oder flüssigen Ablagerung an der Lichteintrittsfläche 11 unterbindet.

Figur 11 zeigt eine schematische Darstellung eines Querschnitts durch das anhand der Figuren 8 und 9 dargestellte Endoskopiesystem oder durch ein Endoskopiesystem mit dem anhand der Figur 10 dargestellten Reinigungsinstrument entlang der in den Figuren 9 und 10 angedeuteten Schnittebene A-A.

Der Querschnitt des mittleren Schaftabschnitts 30 und der Querschnitt des Instrumentenschafts 63 des Reinigungsinstruments 60 (vgl. Figuren 8 bis 10) sind jeweils ohne innere Struktur dargestellt. Der distale Schaftabschnitt 20 des Endoskops 10 (vgl. Figuren 1 und 8 bis 10) liegt vor der Schnittebene A-A und ist deshalb in Figur 11 nicht sichtbar. Um die Position des distalen Schaftabschnitts 20 relativ zu dem mittleren Schaftabschnitt 30 und relativ zu dem proximalen Schaftabschnitt 40 sichtbar zu machen, ist der Rand 21 des Querschnitts des distalen Schaftabschnitts 20 in Figur 11 durch eine gestrichelte Linie angedeutet. Dies entspricht einer Projektion in einer Richtung parallel zu den Längsachsen 28, 38, 48, 58, 68, 88 (vgl. Figuren 1 bis 5).

Der Rand 21 des Querschnitts des distalen Schaftabschnitts, der Rand 31 des Querschnitts des mittleren Schaftabschnitts 30, der Rand 41 des Querschnitts des proximalen Schaftabschnitts 40 sind jeweils kreisförmig, der Querschnitt des Trokartubus 80 ist kreisringförmig. Der distale Schaftabschnitt 20, der mittlere Schaftabschnitt 30 und der proximale Schaftabschnitt 40 sind relativ zueinander so angeordnet, dass der Querschnitt des mittleren Schaftabschnitts 30 am Rand des Querschnitts des distalen Schaftabschnitts 20 und am Rand 41 des Querschnitts des proximalen Schaftabschnitts 40 liegt. Dabei sind die Querschnitte des distalen Schaftabschnitts 20 und des proximalen Schaftabschnitts 40 relativ zu dem mittleren Schaftabschnitt 30 so versetzt, dass der distale Schaftabschnitt 20 und der proximale Schaftabschnitt 40 relativ zu dem mittleren Schaftabschnitt 30 in entgegengesetzte Richtungen überstehen.

Figur 12 zeigt eine schematische Darstellung eines Querschnitts durch ein weiteres Endoskopiesystem, das in einigen Merkmalen und Eigenschaften und Funktionen den anhand der Figuren 1 bis 11 dargestellten Endoskopiesystemen ähnelt. Die Schnittebene der Figur 12 entspricht der Schnittebene A-A der Figur 11. Nachfolgend sind insbesondere Merkmale, Eigenschaften und Funktionen des in Figur 12 gezeigten Endoskopiesystems beschrieben, in denen dieses sich von den anhand der Figuren 1 bis 11 dargestellten Endoskopiesystemen unterscheidet.

Das in Figur 12 gezeigte Endoskopiesystem unterscheidet sich von den anhand der Figuren 1 bis 11 dargestellten Endoskopiesystemen insbesondere dadurch, dass der mittlere Schaftabschnitt 30 des Endoskops keinen kreisförmigen Querschnitt aufweist. Stattdessen weist der mittlere Schaftabschnitt 30 einen Rand auf, der aus zwei jeweils kreisbogenförmigen Randabschnitten 31, 32 gebildet ist. Die kreisbogenförmigen Randabschnitte 31, 32 weisen jeweils den gleichen Radius auf wie der Rand 21 des Querschnitts des distalen Schaftabschnitts 20 und der Rand 41 des Querschnitts des proximalen Schaftabschnitts 40.

Der distale Schaftabschnitt 20, der mittlere Schaftabschnitt 30 und der proximale Schaftabschnitt 40 sind so angeordnet, dass in der in Figur 12 dargestellten Projektion der erste kreisbogenförmige Randabschnitt 31 des Querschnitts des mittleren Schaftabschnitts 30 sich mit dem Rand 21 des distalen Schaftabschnitts 20 deckt, und dass der zweite kreisbogenförmige Randabschnitt 32 des mittleren Schaftabschnitts 30 sich mit dem Rand 41 des proximalen Schaftabschnitts 40 deckt. Somit fluchtet ein Teil der Oberfläche des mittleren Schaftabschnitts 30 mit einem Teil der Oberfläche des distalen Schaftabschnitts 20 und geht glatt in diese über. Ein anderer Teil der Oberfläche des mittleren Schaftabschnitts 30 fluchtet mit einem Teil der Oberfläche des proximalen Schaftabschnitts 40 und geht glatt in diese über.

Figur 13 zeigt eine schematische Darstellung eines Querschnitts durch ein weiteres Endoskopiesystem, das in einigen Merkmalen und Eigenschaften und Funktionen den anhand der Figuren 1 bis 11 dargestellten Endoskopiesystemen und insbesondere dem anhand der Figur 12 dargestellten Endoskopiesystem ähnelt. Die Schnittebene der Figur 13 entspricht der Schnittebene A-A der Figur 11 und der Schnittebene der Figur 12. Nachfolgend sind Merkmale, Eigenschaften und Funktionen des in Figur 13 gezeigten Endoskopiesystems beschrieben, in denen dieses sich von den anhand der Figuren 1 bis 12 dargestellten Endoskopiesystemen unterscheidet.

Das in Figur 13 gezeigte Endoskopiesystem unterscheidet sich von den anhand der Figuren 1 bis 12 dargestellten Endoskopiesystemen und vor allem von dem anhand der Figur 12 dargestellten Endoskopiesystem unter anderem dadurch, dass der Querschnitt des Arbeitskanals 50 in dem proximalen Schaftabschnitt 40 und der dazu korrespondierende Querschnitt des Instrumentenschafts 63 jeweils mondförmig ausgebildet sind. Die Ränder der Querschnitte des Arbeitskanals 50 und des Instrumentenschafts 63 sind jeweils im Wesentlichen aus zwei kreisbogenförmigen Randabschnitten zusammengesetzt. Der Radius des einen Kreisbogenabschnitts ist etwas größer und der Radius des anderen Kreisbogenabschnitts ist etwas kleiner als die Radien der Ränder bzw. Randabschnitte 21, 31, 32, 41 der Querschnitte der Schaftabschnitte 20, 30, 40. Der Querschnitt des mittleren Schaftabschnitts 30 und der Querschnitt des Instrumentenschafts 63 füllen zusammen den Querschnitt des Lumens 86 des Trokartubus 80 weitgehend - insbesondere bis auf eine konstruktiv notwendige Wandstärke des proximalen Schaftabschnitts 40 - aus.

Das in Figur 13 gezeigte Endoskopiesystem unterscheidet sich von den anhand der Figuren dargestellten Endoskopiesystemen ferner dadurch, dass der Instrumentenschaft 63 an dem mittleren Schaftabschnitt 30 und optional auch an dem distalen Schaftabschnitt formschlüssig geführt ist. Dazu weisen der mittlere Schaftabschnitt 30 eine Nut 93 und der Instrumentenschaft 63 einen Steg 96 auf. Die Nut 93 und der Steg 96 weisen korrespondierende T-förmige Querschnitte auf. Der Steg 96 greift in die Nut 93. Formschluss zwischen der Nut 93 und dem Steg 96 unterbindet eine Bewegung des Instrumentenschafts 63 relativ zu dem mittleren Schaftabschnitt 30 weitgehend oder bis auf unvermeidliches Spiel vollständig.

Alternativ zu T-förmigen Querschnitten können die Nut 93 und der Steg 96 schwalbenschwanzförmige Querschnitte aufweisen.

Ein distales Ende der Nut 93 und ein distales Ende des Stegs 96 können einen mechanischen Anschlag bilden, der eine Arbeits- bzw. Reinigungsposition (vgl. Figuren 9, 10) formschlüssig definiert.

Figur 14 zeigt eine schematische Darstellung eines Querschnitts durch ein weiteres Endoskopiesystem, das in einigen Merkmalen, Eigenschaften und Funktionen den anhand der Figuren 1 bis 13 dargestellten Endoskopiesystemen ähnelt. Die Schnittebene der Figur 14 entspricht den Schnittebenen der Figur 11 bis 13. Nachfolgend sind insbesondere Merkmale, Eigenschaften und Funktionen des in Figur 14 gezeigten Endoskopiesystems beschrieben, in denen dieses sich von den anhand der Figuren 1 bis 13 dargestellten Endoskopiesystemen unterscheidet.

Das in Figur 14 gezeigte Endoskopiesystem unterscheidet sich von den anhand der Figuren 1 bis 12 dargestellten Endoskopiesystemen und insbesondere von dem anhand der Figur 13 dargestellten Endoskopiesystem unter anderem dadurch, dass der Querschnitt des mittleren Schaftabschnitts 30 und der Querschnitt des Instrumentenschafts 63 zusammen das Lumen 86 des Trokartubus 80 im Wesentlichen vollständig ausfüllen. Dazu kann der proximale Schaftabschnitt 40 (vgl. Figuren 1 und 5 bis 13) entfallen. In diesem Fall ist das Endoskop nur dann spielarm in dem Trokartubus 80 geführt, wenn gleichzeitig der Instrumentenschaft 63 in den Arbeitskanal 50 in der Handhabungseinrichtung 14 des Endoskops 10 (vgl Figuren 1 und 5 bis 10) und zumindest teilweise in das Lumen 86 des Trokartubus 80 eingeführt ist.

Alternativ kann das Lumen 86 des Trokartubus 80 abweichend von der Darstellung in den Figuren 3 bis 10 nahe dem proximalen Ende 81 einen erweiterten Querschnitt aufweisen, um einen entsprechend vergrößerten proximalen Schaftabschnitt 40 aufzunehmen.

Das in Figur 14 gezeigte Endoskopiesystem unterscheidet sich von den anhand der Figuren 1 bis 13 dargestellten Endoskopiesystemen und insbesondere von dem anhand der Figur 13 dargestellten Endoskopiesystem ferner dadurch, dass eine formschlüssige Führung des Instrumentenschafts 63 an dem mittleren Schaftabschnitt 30 des Endoskops auf andere Weise erzeugt wird. Der mittlere Schaftabschnitt 30 weist an seinen beiden voneinander abgewandten Kanten jeweils eine Nut 93 auf. In jede der beiden Nuten 93 greift ein zugeordneter L-förmiger Steg 96 an dem Instrumentenschaft 63 ein.

Figur 15 zeigt ein schematisches Flussdiagramm eines Verfahrens zum Vorbereiten eines Endoskopiesystems für eine Verwendung. Das Verfahren ist auch für Endoskopiesysteme verwendbar bzw. mit Endoskopiesystemen ausführbar, die sich von den anhand der Figuren 1 bis 14 dargestellten unterscheiden. Trotzdem werden nachfolgend Bezugszeichen aus den Figuren 1 bis 14 verwendet, um das Verständnis zu vereinfachen.

Bei einem ersten Schritt 101 wird ein Endoskop 10 in ein Lumen 86 eines Trokartubus 80 bis zu einer vorbestimmten Position eingeführt. Diese vorbestimmte Position entspricht insbesondere der anhand der Figur 5 dargestellten Position.

Bei einem zweiten Schritt 102 wird das Endoskop 10 in dem Lumen 86 des Trokartubus 80 und relativ zu dem Trokartubus in einer Richtung orthogonal zu der Bewegungsrichtung des ersten Schritts 101 und orthogonal zu den Längsachsen 28, 38, 48 des Endoskops 10 und zu der Längsachse 88 des Trokartubus 80 bewegt. Bei dem zweiten Schritt 102 wird das Endoskop 10 insbesondere von der anhand der Figur 5 dargestellten Position zu der anhand der Figur 6 dargestellten Position relativ zu dem Trokartubus 80 bewegt.

Bei einem optionalen dritten Schritt 103 wird das Endoskop 10 bis zu einer Arbeitsposition relativ zu dem Trokartubus bewegt. Die Bewegungsrichtung des dritten Schritts 103 ist insbesondere parallel zu der Bewegungsrichtung des ersten Schritts 101 und zu den Längsachsen 28, 38, 48 des Endoskops 10 und zu der Längsachse 88 des Trokartubus 80. Bei dem dritten Schritt kann ein proximaler Schaftabschnitt 40 in das Lumen 86 des Trokartubus 80 eingeführt werden. Der dritte Schritt 103 kann insbesondere dann entfallen, wenn der proximale Schaftabschnitt 40 des Endoskops 10 nicht in das Lumen 86 des Trokartubus 80 eingeführt werden soll oder wenn das Endoskop 10 keinen proximalen Schaftabschnitt 40 aufweist.

Bei einem optionalen vierten Schritt 104 wird ein Instrument, beispielweise ein Reinigungsinstrument 60 teilweise durch einen Arbeitskanal 50 des Endoskops 10 hindurch geführt. Die Bewegungsrichtung des vierten Schritts 104 entspricht insbesondere den Bewegungsrichtungen des ersten Schritts 101 und des dritten Schritts 103 und ist parallel zu einer Längsachse 58 des Arbeitskanals 50, parallel zu einer Längsachse 68 des Instruments 60 und insbesondere auch parallel zu einer Längsachse 38 eines Schaftabschnitts 30 des Endoskops 10.

Bei einem fünften Schritt 105 wird das Instrument 60 teilweise an einem Schaftabschnitt, insbesondere an einem mittleren Schaftabschnitt 30 des Endoskops 10 vorbei geführt. Die Bewegungsrichtung des fünften Schritts 105 entspricht der Bewegungsrichtung des vierten Schritts 104 und ist insbesondere parallel zu einer Längsachse 38 eines Schaftabschnitts 30 des Endoskops 10. Der fünfte Schritt 105 kann sich an den vierten Schritt 104 unmittelbar anschließen, so dass der vierte Schritt 104 in den fünften Schritt 105 übergeht. Am Ende des fünften Schritts 105 wird eine Arbeits- bzw. Reinigungsposition des Instruments 60 erreicht.

Wenn das Instrument 60 ein Reinigungsinstrument ist, kann bei einem sechsten Schritt 106 eine Lichteintrittsfläche 11 des Endoskops 10 gereinigt werden. Dazu werden beispielsweise Ablagerungen mittels einer über die Lichteintrittsfläche 11 bewegten Wischlippe 72 von der Lichteintrittfläche 11 abgestreift oder mittels eines von einer Düse 74 geformten Fluidstroms 75 von der Lichteintrittsfläche 11 gespült. Alternativ kann bei dem sechsten Schritt 106 ein Fluidstrom 75 an der Lichteintrittsfläche 11 des Endoskops 10 erzeugt werden, der die Entstehung von Ablagerungen verhindert.

Bezugszeichen
- 10: Endoskop
- 11: Lichteintrittsfläche an dem distalen Ende 12 des Endoskops 10
- 12: distales Ende des Endoskops 10
- 13: Endoskopschaft des Endoskops 10
- 14: Handhabungseinrichtung am proximalen Ende 15 des Endoskops 10
- 15: proximales Ende des Endoskops 10
- 20: distaler Schaftabschnitt des Schafts 13 des Endoskops 10
- 21: Rand des Querschnitts des distalen Schaftabschnitts 20
- 28: Längsachse des distalen Schaftabschnitts 20
- 30: mittlerer Schaftabschnitt des Schafts 13 des Endoskops 10
- 31: erster Randabschnitt des Querschnitts des mittleren Schaftabschnitts 30
- 32: zweiter Randabschnitt des Querschnitts des mittleren Schaftabschnitts 30
- 34: proximales Ende des mittleren Schaftabschnitts 30
- 38: Längsachse des mittleren Schaftabschnitts 30
- 40: proximaler Schaftabschnitt des Schafts 13 des Endoskops 10
- 41: Rand des Querschnitts des proximalen Schaftabschnitts 40
- 48: Längsachse des proximalen Schaftabschnitts 40
- 50: Arbeitskanal in dem proximalen Schaftabschnitts 40 und in der Handhabungseinrichtung 14 des Endoskops 10 zur Aufnahme eines Instruments 60
- 52: Dichteinrichtung in dem Arbeitskanal 50 zum fluiddichten Abdichten des Arbeitskanals 50 oder zum Verringern seines Querschnitts
- 53: Austrittsöffnung des Arbeitskanals 50 am distalen Ende des proximalen Schaftabschnitts 40
- 58: Längsachse des Arbeitskanals 50
- 60: Reinigungsinstrument zum Reinigen der Lichteintrittfläche 11 des Endoskops 10
- 61: distales Ende des Reinigungsinstruments 60
- 63: Instrumentenschaft des Reinigungsinstruments 60
- 64: Rad an dem Reinigungsinstrument 60, das proximale Ende des Reinigungsinstruments 60 bildend
- 68: Längsachse des Instrumentenschafts 63
- 70: Reinigungswerkzeug am distalen Ende 61 des Reinigungsinstruments 60

- 72: Wischlippe des Reinigungswerkzeugs 70 zum Abstreifen von Ablagerungen auf der Lichteintrittsfläche 11 des Endoskops 10
- 73: Fluidkanal in dem Instrumentenschaft 63
- 74: Düse des Reinigungswerkzeugs 70
- 75: von der Düse 74 geformter Fluidstrom an der Lichteintrittsfläche 11 des Endoskops
- 76: Gelenk zwischen dem Reinigungswerkzeug 70 und dem Instrumentenschaft 63 des Reinigungsinstruments 60
- 78: Schwenkachse des Gelenks 76
- 80: Trokartubus
- 81: proximales Ende des Trokartubus 80
- 82: distales Ende des Trokartubus 80
- 86: Lumen des Trokartubus
- 88: Längsachse des Trokartubus 80
- 93: Nut als Führungseinrichtung an dem Endoskopschaft 13 des Endoskops 10
- 96: zu der Nut 93 korrespondierender Steg als Führungseinrichtung an dem Reinigungsinstrument 60
- 101: erster Schritt (Einführen eines Endoskops in einen Trokartubus bis zu einer vorbestimmten Position)
- 102: zweiter Schritt (Bewegen des Endoskops in dem Trokartubus in einer Richtung orthogonal zu der Einführrichtung)
- 103: dritter Schritt (Bewegen des Endoskops bis zu einer Arbeitsposition)
- 104: vierter Schritt (Hindurchführen eines Instruments durch einen Arbeitskanal des Endoskops)
- 105: fünfter Schritt (Vorbeiführen des Instruments an einem Schaftabschnitt des Endoskops)
- 106: sechster Schritt (Reinigen einer Lichteintrittsfläche des Endoskops)

## Patentansprüche

1. **Endoskop** (10), mit:
einem **Endoskopschaft** (13) mit einem distalen Schaftabschnitt (20), einem mittleren Schaftabschnitt (30) und einem proximalen Schaftabschnitt (40);
einer **Lichteintrittsfläche** (11) an dem distalen Schaftabschnitt (20),
wobei der **Querschnitt** des mittleren Schaftabschnitts (30) **kleiner** als der Querschnitt des distalen Schaftabschnitts (20) ist,
wobei der Querschnitt des distalen Schaftabschnitts (20) gegenüber dem Querschnitt des mittleren Schaftabschnitts (30) in einer ersten Richtung seitlich versetzt ist,
**dadurch gekennzeichnet, dass**
der Querschnitt des mittleren Schaftabschnitts (30) kleiner als
der Querschnitt des proximalen Schaftabschnitts (40) ist, wobei der Querschnitt des proximalen Schaftabschnitts (40) gegenüber dem Querschnitt des mittleren Schaftabschnitts (30) in einer zweiten Richtung seitlich versetzt ist,
wobei die erste Richtung und die zweite Richtung einander entgegengesetzt oder voneinander verschieden sind.

2. Endoskop (10) nach dem vorangehenden Anspruch, bei dem
der Rand (21) des Querschnitts des distale Schaftabschnitts (20) **kreisförmig** ist und einen ersten Radius aufweist,
der Rand des Querschnitts des mittleren Schaftabschnitts (30) **zwei kreisbogenförmige Randabschnitte** (31, 32) aufweist,
die Radien der kreisbogenförmigen Randabschnitte (31, 32) jeweils gleich dem ersten Radius oder kleiner als der erste Radius sind,
die Mittelpunkte der kreisbogenförmigen Randabschnitte (31, 32) von einander beabstandet sind.

3. Endoskop (10) nach einem der vorangehenden Ansprüche, bei dem
bezogen auf eine Projektion in Richtung parallel zur Längsachse (38) des mittleren Schaftabschnitts (30) der Querschnitt des proximalen Schaftabschnitts (40) und der Querschnitt des distalen Schaftabschnitts (20) gegenüber dem Querschnitt des mittleren Schaftabschnitts (30) **in entgegengesetzten Richtungen überstehen.**

4. Endoskop (10) nach einem der vorangehenden Ansprüchen, bei dem der mittlere Schaftabschnitt (30) **an einer Seite glatt in den distalen Schaftabschnitt** (20) übergeht und an einer **entgegengesetzten Seite glatt in den proximalen Schaftabschnitt** (40) übergeht.

5. Endoskop (10) nach einem der vorangehenden Ansprüche, bei dem bezogen auf eine Projektion in Richtung parallel zur Längsachse (38) des mittleren Schaftabschnitts (30) der Querschnitt des mittleren Schaftabschnitts (30) an einem Abschnitt des Rands (41) des Querschnitts des proximalen Schaftabschnitts (40) und an einem gegenüberliegenden Abschnitt des Rands (21) des Querschnitts des distalen Schaftabschnitts (20) angeordnet ist.

6. Endoskop (10) nach einem der einem der vorangehenden Ansprüche, ferner mit:
einem **Arbeitskanal** (50) zur Aufnahme eines Instruments (60) in dem proximalen Schaftabschnitt (40),
wobei eine distale Austrittsöffnung (53) des Arbeitskanals (50) neben dem proximalen Ende (34) des mittleren Schaftabschnitts (30) angeordnet ist.

7. Endoskop (10) nach dem vorangehenden Anspruch, bei dem
der Querschnitt des Arbeitskanals (50) in dem proximalen Schaftabschnitt (40) **nicht kreisförmig** ist.

8. Endoskop (10) nach dem vorangehenden Anspruch, bei dem
der **Querschnitt des Arbeitskanals** (50) den Querschnitt des proximalen Schaftabschnitts (40) **abzüglich des Querschnitts des mittleren Schaftabschnitts** (30) weitgehend ausfüllt.

9. Endoskop (10) nach einem der vorangehenden Ansprüche, ferner mit:
einer **Führungseinrichtung** (93) zum Führen eines Instruments (60) an dem mittleren Schaftabschnitt (30).

10. **Endoskopiesystem** (10, 80) mit:
einem **Endoskop** (10) nach einem der Ansprüche 1 bis 9;
einem **Trokartubus** (80) mit einem Lumen (88) zur Aufnahme des Endoskopschafts (13) des Endoskops (10),
wobei der Endoskopschaft (13) des Endoskops (10) und der Trokartubus (80) so ausgebildet sind, dass der distale Schaftabschnitt (20) des Endoskops (10) vollständig durch den Trokartubus (80) hindurchgeführt werden kann.

11. Endoskopiesystem (10, 80) nach dem vorangehenden Anspruch, bei dem
der **Trokartubus** (80) oder ein zylindrischer Abschnitt des Lumens (88) des Trokartubus (80) **nicht länger** als der **mittlere Schaftabschnitt** (30) des Endoskops (10) ist.

12. **Endoskopiesystem** (10, 80) nach einem der Ansprüche 10 und 11, ferner mit einem **Reinigungsinstrument** (60) zur Reinigung oder Trocknung der Lichteintrittsfläche (11) an dem distalen Schaftabschnitt (20) des Endoskops (10), wobei das Reinigungsinstrument (60) umfasst:
einen **Instrumentenschaft** (63);
ein **Werkzeug** (70) an einem distalen Ende des Instrumentenschafts (63) zum Reinigen oder Trocknen der Lichteintrittsfläche (11) an dem distalen Schaftabschnitt (20) des Endoskops (10),
wobei der Instrumentenschaft (63) zur Anordnung neben dem Endoskopschaft (13) des Endoskops (10) vorgesehen und ausgebildet ist,
wobei der mittlere Schaftabschnitt (30) des Endoskops (10), der Instrumentenschaft (63) des Reinigungsinstruments (60) und der Trokartubus (80) dafür vorgesehen und ausgebildet sind, dass der Querschnitt des mittleren Schaftabschnitts (30) des Endoskops (10) und
der Querschnitt des Instrumentenschafts (63) des Reinigungsinstruments (60) den Querschnitt des Lumens (88) des Trokartubus (80) weitgehend ausfüllen.

13. Endoskopiesystem (10, 80) nach dem vorangehenden Anspruch, bei dem
der Querschnitt des Instrumentenschafts (63) so an den Querschnitt des mittleren Schaftabschnitts (30) des Endoskops (10) angepasst ist, dass der **Gesamtquerschnitt einer parallelen Anordnung** des Instrumentenschafts (63) und des mittleren Schaftabschnitts (30) des Endoskops (10) nicht größer ist als der Querschnitt des distalen Schaftabschnitts (20) des Endoskops (10).

14. Endoskopiesystem (10, 80) nach einem der Ansprüche 12 und 13, ferner mit:
einem **Fluidkanal** (73) in dem Instrumentenschaft (63),
wobei das Werkzeug (70) eine **Düse** (74), die mit dem distalen Ende des Fluidkanals (73) verbunden ist, umfasst,
wobei die Düse (74) zum Leiten eines Fluids zu der Lichteintrittsfläche (11) des Endoskops (10) vorgesehen und ausgebildet ist.

15. Endoskopiesystem (10, 80) nach einem der Ansprüche 12 bis 14, ferner mit:
einer **Führungseinrichtung** (96) zum Führen des Reinigungsinstruments (60) an einem Endoskopschaft (13) des Endoskops (10).

16. Endoskopiesystem (10, 80) nach einem der Ansprüche 12 bis 15, bei dem
das Werkzeug (70) ein **Gelenk** (76) aufweist, das ein Anlegen einer Wischlippe (72) an die Lichteintrittsfläche (11) des Endoskops (10) ermöglicht.

## Claims

1. Endoscope (10) comprising:
an endoscope shaft (13), with a distal shaft portion (20), a central shaft portion (30) and a proximal shaft portion (40);
a light admission face (11) at the distal shaft portion (20),
wherein the cross section of the central shaft portion (30) is smaller than the cross section of the distal shaft portion (20),
wherein the cross section of the distal shaft portion (20) is laterally offset in a first direction with respect to the cross section of the central shaft portion (30),
**characterized in that**
the cross section of the central shaft portion (30) is smaller than the cross section of the proximal shaft portion (40),
wherein the cross section of the proximal shaft portion (40) is laterally offset in a second direction with respect to the cross section of the central shaft portion (30),
wherein the first direction and the second direction are counter to each other or different than each other.

2. Endoscope (10) according to the preceding claim, wherein
the edge (21) of the cross section of the distal shaft portion (20) is circular and has a first radius,
the edge of the cross section of the central shaft portion 30) has two circular-arc-shaped edge portions(31, 32),
the radii of the circular-arc-shaped edge portions (31, 32) are each equal to the first radius or smaller than the first radius,
the center points of the circular-arc-shaped edge portions (31, 32) are spaced apart from each other.

3. Endoscope (10) according to one of the preceding claims, wherein,
in relation to a projection in a direction parallel to the longitudinal axis (38) of the central shaft portion (30), the cross section of the proximal shaft portion (40) and the cross section of the distal shaft portion (20) protrude in opposite directions with respect to the cross section of the central shaft portion (30).

4. Endoscope (10) according to one of the preceding claims, wherein the central shaft portion (30) merges at one side smoothly into the distal shaft portion (20) and merges at an opposite side smoothly into the proximal shaft portion (40).

5. Endoscope (10) according to one of the preceding claims, wherein, in relation to a projection in a direction parallel to the longitudinal axis (38) of the central shaft portion (30), the cross section of the central shaft portion (30) is arranged at a portion of the edge (41) of the cross section of the proximal shaft portion (40) and at an opposite portion of the edge (21) of the cross section of the distal shaft portion (20).

6. Endoscope (10) according to one of the preceding claims, further comprising:
a working channel (50) for receiving an instrument (60) in the proximal shaft portion (40),
wherein a distal outlet opening (53) of the working channel (50) is arranged beside the proximal end (34) of the central shaft portion (30).

7. Endoscope (10) according to the preceding claim, wherein
the cross section of the working channel (50) in the proximal shaft portion (40) is not circular.

8. Endoscope (10) according to the preceding claim, wherein
the cross section of the working channel (50) substantially fills the cross section of the proximal shaft portion (40), less the cross section of the central shaft portion (30).

9. Endoscope (10) according to one of the preceding claims, further comprising:
a guiding mechanism (93) for guiding an instrument (60) at the central shaft portion (30).

10. Endoscopy system (10, 80) comprising:
an endoscope (10) according to one of the claims 1 through 9;
a trocar tube (80) with a lumen (88) for receiving the endoscope shaft (13) of the endoscope (10),
wherein the endoscope shaft (13) of the endoscope (10) and the trocar tube (80) are designed such that the distal shaft portion (20) of the endoscope (10) can be guided completely through the trocar tube (80).

11. Endoscopy system (10, 80) according to the preceding claim, wherein the trocar tube (80) or a cylindrical portion of the lumen (88) of the trocar tube (80) is not longer than the central shaft portion (30) of the endoscope (10).

12. Endoscopy system (10, 80) according to one of the claims 10 and 11, further comprising a cleaning instrument (60) for cleaning or drying the light admission face (11) at the distal shaft portion (20) of an endoscope (10), the cleaning instrument (60) comprising:
an instrument shaft (63);
a tool (70) at the distal end of the instrument shaft (63) for cleaning or drying the light admission face (11) at the distal shaft portion (20) of the endoscope (10),
wherein the instrument shaft (63) is provided and designed to be arranged beside an endoscope shaft (13) of the endoscope (10),
wherein the central shaft portion (30) of the endoscope (10), the instrument shaft (63) of the cleaning instrument (60) and the trocar tube (80) are provided and designed such that the cross section of the central shaft portion (30) of the endoscope (10) and the cross section of the instrument shaft (63) of the cleaning instrument (60) substantially fill the cross section of the lumen (88) of the trocar tube (80).

13. Endoscopy system (10, 80) according to the preceding claim, wherein
the cross section of the instrument shaft (63) is adapted to the cross section of the central shaft portion (30) of the endoscope (10) such that the total cross section of a parallel arrangement of the instrument shaft (63) and of the central shaft portion (30) of the endoscope (10) is not greater than the cross section of the distal shaft portion (20) of the endoscope (10).

14. Endoscopy system (10, 80) according to one of the claims 12 and 13, further comprising:
a fluid channel (73) in the instrument shaft (63),
wherein the tool (70) comprises a nozzle (74), which is connected to the distal end of the fluid channel (73),
wherein the nozzle (74) is provided and designed to convey a fluid to the light admission face (11) of the endoscope (10).

15. Endoscopy system (10, 80) according to one of the claims 12 through 14, further comprising:
a guiding mechanism (96) for guiding the cleaning instrument (60) at an endoscope shaft (13) of the endoscope (10).

16. Endoscopy system (10, 80) according to one of the claims 12 through 15, wherein
the tool (70) has a hinge (76), which facilitates placement of a wiping lip (72) at the light admission face (11) of the endoscope (10).

## Revendications

1. Endoscope (10) comprenant :
une tige d'endoscope (13) pourvue d'une portion de tige distale (20), d'une portion de tige médiane (30) et d'une portion de tige proximale (40) ;
une surface d'entrée de lumière (11) au niveau de la portion de tige distale (20),
la section transversale de la portion de tige médiane (30) étant plus petite que la section transversale de la portion de tige distale (20),
la section transversale de la portion de tige distale (20) étant décalée latéralement dans une première direction par rapport à la section transversale de la portion de tige médiane (30),
**caractérisé en ce que**
la section transversale de la portion de tige médiane (30) est plus petite que la section transversale de la portion de tige proximale (40),
la section transversale de la portion de tige proximale (40) étant décalée latéralement dans une deuxième direction par rapport à la section transversale de la portion de tige médiane (30),
la première direction et la deuxième direction étant opposées l'une à l'autre ou
différentes l'une de l'autre.

2. Endoscope (10) selon la revendication précédente, dans lequel le bord (21) de la section transversale de la portion de tige distale (20) est circulaire et possède un premier rayon,
le bord de la section transversale de la portion de tige médiane (30) comporte deux portions de bord incurvées (31, 32),
les rayons des portions de bord incurvées (31, 32) sont chacun égaux au premier rayon ou inférieurs au premier rayon,
les centres des portions de bord incurvées (31, 32) sont espacés les uns des autres.

3. Endoscope (10) selon l'une des revendications précédentes, dans lequel
sur la base d'une projection dans la direction parallèle à l'axe longitudinal (38) de la portion de tige médiane (30) la section transversale de la portion de tige proximale (40) et la section transversale de la portion de tige distale (20) font saillie dans des directions opposées par rapport à la section transversale de la portion de tige médiane (30).

4. Endoscope (10) selon l'une des revendications précédentes, dans lequel la portion de tige médiane (30) se transforme sur un côté tout simplement en la portion de tige distale (20) et se transforme sur un côté opposé tout simplement en la portion de tige proximale (40).

5. Endoscope (10) selon l'une des revendications précédentes, dans lequel sur la base d'une projection dans la direction parallèle à l'axe longitudinal (38) de la portion de tige médiane (30) la section transversale de la portion de tige médiane (30) est disposée au niveau d'une portion du bord (41) de la section transversale de la portion de tige proximale (40) et au niveau d'une portion opposée du bord (21) de la section transversale de la portion de tige distale (20).

6. Endoscope (10) selon l'une des revendications précédentes, comprenant en outre :
un conduit de travail (50) destiné à recevoir un instrument (60) dans la portion de tige proximale (40),
une ouverture de sortie distale (53) du conduit de travail (50) étant ménagée à côté de l'extrémité proximale (34) de la portion de tige médiane (30).

7. Endoscope (10) selon la revendication précédente, dans lequel
la section transversale du conduit de travail (50) dans la portion de tige proximale (40) n'est pas circulaire.

8. Endoscope (10) selon la revendication précédente, dans lequel
la section transversale du conduit de travail (50) correspond dans une large mesure à la section transversale de la portion de tige proximale (40) moins la section transversale de la portion de tige médiane (30).

9. Endoscope (10) selon l'une des revendications précédentes, comprenant en outre :
un dispositif de guidage (93) destiné à guider un instrument (60) sur la portion de tige médiane (30).

10. Système d'endoscopie (10, 80) comprenant :
un endoscope (10) selon l'une des revendications 1 à 9 ;
un tube de trocart (80) pourvu d'une lumière (88) destiné à recevoir la tige d'endoscope (13) de l'endoscope (10),
la tige d'endoscope (13) de l'endoscope (10) et le tube de trocart (80) étant conçus de telle sorte que la portion de tige distale (20) de l'endoscope (10) puisse être entièrement guidée à travers le tube de trocart (80).

11. Système d'endoscopie (10, 80) selon la revendication précédente, dans lequel
le tube de trocart (80) ou une portion cylindrique de la lumière (88) du tube de trocart (80) n'est pas plus long que la portion de tige médiane (30) de l'endoscope (10).

12. Système d'endoscopie (10, 80) selon l'une des revendications 10 et 11, comprenant en outre un instrument de nettoyage (60) destiné à nettoyer ou sécher la surface d'entrée de lumière (11) sur la portion de tige distale (20) de l'endoscope (10), l'instrument de nettoyage (60) comprenant :
une tige d'instrument (63) ;
un outil (70) placé à une extrémité distale de la tige d'instrument (63) et destiné à nettoyer ou sécher la surface d'entrée de lumière (11) sur la portion de tige distale (20) de l'endoscope (10),
la tige d'instrument (63) étant prévue et conçue pour être disposée à côté de la tige d'endoscope (13) de l'endoscope (10),
la portion de tige médiane (30) de l'endoscope (10), la tige d'instrument (63) de l'instrument de nettoyage (60) et le tube de trocart (80) étant prévus et conçus de sorte que la section transversale de la portion de tige médiane (30) de l'endoscope (10) et la section transversale de la tige d'instrument (63) de l'instrument de nettoyage (60) correspondent dans une large mesure à la section transversale de la lumière (88) du tube de trocart (80).

13. Système d'endoscopie (10, 80) selon la revendication précédente, dans lequel
la section transversale de la tige d'instrument (63) est adaptée à la section transversale de la portion de tige médiane (30) de l'endoscope (10) de telle sorte que la section transversale totale d'un agencement parallèle de la tige d'instrument (63) et de la portion de tige médiane (30) de l'endoscope (10) n'est pas supérieure à la section transversale de la portion de tige distale (20) de l'endoscope (10).

14. Système d'endoscopie (10, 80) selon l'une des revendications 12 et 13, comprenant en outre :
un conduit de fluide (73) dans la tige d'instrument (63),
l'outil (70) comprenant une buse (74) qui est reliée à l'extrémité distale du conduit de fluide (73),
la buse (74) étant prévue et conçue pour guider un fluide vers la surface d'entrée de lumière (11) de l'endoscope (10).

15. Système d'endoscopie (10, 80) selon l'une des revendications 12 à 14, comprenant en outre :
un dispositif de guidage (96) destiné à guider l'instrument de nettoyage (60) sur une tige d'endoscope (13) de l'endoscope (10).

16. Système d'endoscopie (10, 80) selon l'une des revendications 12 à 15, dans lequel
l'outil (70) possède une articulation (76) qui permet de placer une lèvre d'essuyage (72) sur la surface d'entrée de lumière (11) de l'endoscope (10).
